# EUROPEAN PATENT SPECIFICATION

(11) **EP 3 919 907 B1**
(45) Date of publication and mention of the grant of the patent: **08.10.2025**
(21) Application number: 20747789.4
(22) Date of filing: 03.01.2020
(51) Int. Cl.: G01N 33/543

(54) **SINGLE MOLECULE QUANTITATIVE DETECTION METHOD AND DETECTION SYSTEM**
VERFAHREN ZUM QUANTITATIVEN NACHWEIS VON EINZELMOLEKÜLEN UND NACHWEISVERFAHREN
PROGRAMME DE DÉTECTION QUANTITATIVE D'UNE UNIQUE MOLÉCULE ET SYSTÈME DE DÉTECTION ASSOCIÉ

(30) Priority: 30.01.2019 CN 201910091631
(43) Date of publication of application: 08.12.2021
(73) Proprietor: Suzhou Astrabio Technology Co., Ltd., Jiangsu 215000 (CN)
(72) Inventor: GUAN, Zhichao, Suzhou Industrial Park Jiangsu 215000 (CN)
(74) Representative: Comoglio, Elena
(86) International application number: PCT/CN2020/070283
(87) International publication number: WO 2020/156029

(56) References cited:
- WO-A2-02/074988
- WO-A2-2008/052774
- CN-A- 103 940 798
- CN-A- 103 940 989
- CN-A- 105 181 981
- CN-A- 105 358 979
- CN-A- 107 643 399
- CN-A- 109 142 754
- CN-A- 109 164 255
- US-A1- 2005 250 094
- ZHUANGQIANG GAO ET AL: "Enhanced Colorimetric Immunoassay Accompanying with Enzyme Cascade Amplification Strategy for Ultrasensitive Detection of Low-Abundance Protein", SCIENTIFIC REPORTS, vol. 4, 10 February 2014 (2014-02-10), XP055191220, DOI: 10.1038/srep03966

## Description

### CROSS-REFERENCE TO RELATED APPLICATIONS

The present application claims the priority to Chinese Patent Application No. CN201910091631.1 filed on January 30, 2019 before the Chinese National Intellectual Property Office, and entitled "Quantitative single-molecule detection method and detection system' .

### TECHNICAL FIELD

The present application relates to a quantitative detection method and detection system based on single molecule counting, which can be used for quantitative detection of proteins, nucleic acids and small molecules with ultra-high sensitivity.

### BACKGROUND OF THE INVENTION

The content of biomarkers, particularly those associated with major diseases, in human body is closely related to various biological processes. The content of these biomarkers is low in the early stage of diseases, so it is difficult for the traditional methods to achieve the accurate and effective detection and analysis. To achieve the early and accurate diagnosis of biomarkers (such as DNA, RNA, or enzymes), quantitative detection methods with ultra-high-sensitivity are needed.

The detection principle underlying the traditional detection methods (such as chemiluminescence, enzyme-linked immunoassay, or fluorescent immunoassay for protein detection, or fluorescent quantitative PCR for nucleic acid detection) is to detect based on the overall optical signal of the solution, and compare the light intensity signal obtained by detection with a standard curve to achieve the quantitative detection. However, due to the presence of the certain background noise signal in the detection equipment, when the concentration of target molecules in the sample is low to a certain level, the overall light intensity signal of the solution will be submerged by the background noise signal, resulting in a larger deviation in a low value interval, and thus it is difficult to achieve accurate quantitative analysis. Therefore, due to limitations in the principle of detection technologies, it is difficult for these traditional detection methods to achieve the detection and analysis with high-sensitivity.

Single-molecule detection or digital single-molecule detection adopts a completely different detection strategy from that of traditional detection methods. In other words, signal amplification or signal labeling at a single molecule level can be realized by directly labeling the molecules to be detected (proteins, small molecules or nucleic acid molecules) with an enzyme molecule or a fluorescent dye molecule or by amplifying the molecules to be detected. Then, the amplified single-molecule signal is read by a detection device, and the number of the molecules to be detected is directly counted. Finally, the quantitative detection at a single molecule level is realized by correcting the number of molecules to be detected obtained by detection using a standard curve.

Currently, the reported quantitative detection at a single molecule level mainly includes the following two strategies:
(1) The molecules to be detected are directly labeled with a fluorescent molecule or nano material emitting an optical, an electrical or a magnetic signal by single molecule labeling technology, and then the signal is detected by the optical, electrical or magnetic detection device having very high precision, for example, total internal reflection microscope, near-field microscope or Airy spot focusing detection device (i.e., optical device using SMC technology invented by Singulex company, U.S. in Patent Document 1) enabling single molecule fluorescence response, and electronic microscope enabling single nanoparticle detection. Because these detection devices need to be able to identify a signal from several or even a single fluorescent molecular(s), the requirement for the precision of the device is extremely high, resulting in extremely high cost of devices. It seriously limits the application of these technologies in the areas of scientific research and medical diagnosis.
(2) By means of signal amplification, an extremely weak single-molecule signal is amplified to a level that can be easily detected by a device. Currently, digital PCR and single-molecule digital enzyme assay are common. In digital PCR, the nucleic acid molecules to be detected are directly amplified exponentially by PCR, and the signal amplification is realized by a fluorescent probe. In single-molecule digital enzyme assay, an enzyme or a catalytic substrate with high catalytic efficiency is labeled on a molecule to be detected, producing a product molecule with fluorescence characteristics, and thereby realizing the signal amplification. Generally, the signal amplification method raises a low requirement on the detection device and the cost is relatively controllable, so it has a broader application than single molecule fluorescence detection. However, the product signal is low in both digital PCR and single-molecule digital enzyme assay. In order to improve the signal-to-background ratio in the detection, it is usually necessary to disperse the molecules to be detected into droplets that are extremely small volume (nanoliter to picoliter) (see Patent Document 2). This makes the operation of this type of technology cumbersome, requires sophisticated operating skills, and requires an auxiliary device for droplet formation having high-precision.

In addition, although the detection technology for nucleic acids at a single molecule level is currently in rapid development (mainly due to the development of single-molecule PCR technology), it is generally believed in the art that, compared to DNA, the structure of proteins is more complex and difficult to replicate and amplify. Due to the lack of exponential signal amplification methods such as PCR, the sensitivity of existing protein detection methods is not high enough, causing that the single-molecule detection technology of proteins is still basically in blank, let alone achieving the single-molecule detection of proteins using conventional fluorescence microscopes. WO 02/074988 A2 discloses spatially addressable low density molecular arrays and analytical approaches based on single molecule detection techniques.

It can be seen that the existing single molecule detection technologies have the problems of complicated detection system, extremely high precision requirement for detection devices, poor stability of detection reagents, high precision requirements for auxiliary consumables, difficulty to control costs, and inability to apply well in protein detection. These problems have greatly hindered the application of single molecule detection technologies in scientific research and medical diagnostic areas.

The references in the prior art
Patent Document 1: CN101438146A
Patent Document 2: CN102884431A

### Summary of the Invention

### [Problems to be solved by the invention]

In view of the state-of-art, the present application is proposed, to provide a novel single-molecule detection method and single-molecule detection system that can realize single-molecule detection by simple single-molecule signal labeling and have low requirements for optical devices.

### [Means for solving problems]

To solve the above-mentioned problems, the inventors of the present application find, after repeated intensive research, a novel quantitative single-molecule detection method and single-molecule detection system based on imaging, and having high sensitivity and low cost.

One of the technical solutions of the present application is as follows.

A quantitative single-molecule detection and analysis method comprising the following steps:
(1) immobilizing a capture antibody able to bind to a target to a solid-phase carrier, and using the capture antibody to bind to a first site of the target molecule to capture the target molecule in a sample;
(2) adding a detection antibody that binds to a second site of the target molecule, and then adding in-situ signal enhancing nanoparticles able to directly or ndirectly bind to the detection antibody; or
   allowing the detection antibody to bind to the in-situ signal enhancing nanoparticles to form a complex material, and then adding the complex material;
   wherein the in-situ signal enhancing nanoparticles comprise a luminescent material and a nanoparticle carrier, and have a particle size of 180-350 nm, and the luminescent material is a fluorescent material;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation,
   wherein the solid-phase carrier comprises magnetic beads

Preferably, the particle size of the in-situ signal enhancing nanoparticles is 200-350 nm.

Further preferably, the particle size of the in-situ signal enhancing nanoparticles is 220-350 nm, more preferably over 220 nm and below 330 nm, and most preferably over 220 nm and below 300 nm.

The target molecule targeted by the technical solution of the present application includes proteins, polysaccharides or biologically active small molecules.

### Effects of the present application

The quantitative single-molecule detection method of the present application is applicable to the detection of proteins and nucleic acids, especially proteins, with ultra-high sensitivity and extremely low cost. When used in the detection of proteins, the inventors of the present application surprisingly find that in some embodiments, the sensitivity can reach 1 pg/ml; in other embodiments, the sensitivity can reach 100 fg/ml; in some embodiments, the sensitivity can reach 10 fg/ml; and the sensitivity can be up to 1 fg/mL. In addition, by means of the present application, the detection can be realized in a short time (only a few minutes of incubation time) while ensuring high detection sensitivity, thereby shortening the detection time in clinic.

The quantitative single-molecule detection system of the present application is based on a specific detection system (particularly using specific in-situ signal enhancing nanoparticles to label the molecule to be detected) and realizes ultra-high-sensitivity detection by a simple method. The detection sensitivity of the quantitative single-molecule detection system of the present application is far higher than that of the traditional methods, with an improvement of 2-4 orders of magnitude.

Compared with the existing single-molecule detection technologies, the present application breaks the barrier between detection of protein molecules and nucleic acid molecules, can detect protein molecules and nucleic acid molecules by the same device, and particularly can be used in protein detection with ultra-high sensitivity. In addition, by the use of a specific detection system (such as specific in-situ signal enhancing nanoparticles), the system can realize in-situ imaging of molecules to be detected by only a low-cost light sensing element such as CCD or CMOS without expensive equipment such as total internal reflection microscope. It greatly reduces the difficulty of single-molecule detection, the complexity of steps and the precision requirement for devices, and enables the single-molecule detection technology to be applied to scientific research and biomedical detection at a low cost.

### BRIEF DESCRIPTION OF THE DRAWINGS

Fig. 1 is a schematic diagram of a protein detection method according to the present application;
Fig. 2 is a schematic diagram of a nucleic acid detection method according to the present application;
Fig. 3 is a standard curve obtained in Example 1 (the vertical coordinate, i.e., CPN (copy number), is the number of single-molecule signals);
Fig. 4 is a standard curve obtained in Comparative Example 1;
Fig. 5 is a standard curve obtained in Comparative Example 2;
Fig. 6 is a standard curve obtained in Example 2;
Fig. 7 is a standard curve obtained in Example 11;
Fig. 8 is a standard curve obtained in Example 14;
Fig. 9 is a standard curve obtained in Example 15;
Fig. 10 is a standard curve obtained in Example 16;
Fig. 11 is a standard curve obtained in Example 17;
Fig. 12 is a standard curve obtained in Example 18;
Fig. 13 shows the comparison results of the clinical samples in Example 18;
Fig. 14 is a standard curve obtained in Example 19;
Fig. 15 shows the comparison results of the clinical samples in Example 19;
Fig. 16 is a standard curve obtained in Example 20; and
Fig. 17 is a standard curve obtained in Example 22.

### DETAILED DESCRIPTION OF THE INVENTION

### <First Embodiment>

A first embodiment of the present invention is shown below.

A quantitative single-molecule detection and analysis method comprising the following steps:
(1) immobilizing a capture antibody able to bind to a target molecule to a solid-phase carrier, and using the capture antibody to bind to a first site of the target molecule to capture the target molecule in a sample;
(2) adding a detection antibody that binds to a second site of the target molecule, and then adding in-situ signal enhancing nanoparticles able to directly or indirectly bind to the detection antibody; or
   allowing the detection antibody to bind to the in-situ signal enhancing nanoparticles to form a complex material, and then adding the complex material;
   wherein the in-situ signal enhancing nanoparticles comprise a luminescent material and a nanoparticle carrier, and have a particle size of 180-350 nm, and the luminescent material is a fluorescent material;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation,
   wherein the solid-phase carrier comprises magnetic beads.

In the present application, the solid-phase carrier is used for the separation and cleaning of the test sample and the reagent. The capture antibody is immobilized on the surface of the solid-phase carrier by physical adsorption or chemical modification, and can bind to a binding site of the target molecule (hereinafter, sometimes also referred to as "the molecules to be detected") to separate it from the sample.

The detection antibody can bind to another binding site of the molecules to be detected. The in-situ signal enhancing nanoparticles directly bind to the detection antibody, which means that the detection antibody is directly adsorbed or conjugated to the in-situ signal enhancing nanoparticles by physical adsorption or chemical modification to achieve the functional modification for recognition and labeling on the molecules to be detected with the in-situ signal enhancing nanoparticles. The "in-situ signal enhancing nanoparticles indirectly bind to the detection antibody" refers to binding through an anti-detection antibody (i.e. secondary antibody) or a biotin-streptavidin system to specifically label the in-situ signal enhancing nanoparticles on the detection antibody.

The target molecule includes proteins, polysaccharides, or biologically active small molecules and the complexes of small molecules and proteins. Specifically, examples include cTnI antigen, IL-6 antigen, PCT (procalcitonin) antigen, Sema4D (semaphorin 4D) antigen, Nt-proBNP (N terminal pro B type natriuretic peptide) antigen, tumor markers, Vitamin D, vitamin B, folic acid, vitamin D-BSA complex, folic acid-BSA complex, bacteria and viruses.

The solid-phase carrier includes, according to the morphology, magnetic beads, perforated plates, centrifuge tubes, chips, micro-scale microspheres and nano-scale microspheres, etc.; includes, according to the material, polymers, silica, silicon or their complexes; and includes, according to the function, magnetic solid-phase carrier and non-magnetic solid-phase carrier. Among these solid-phase carriers, magnetic beads are particularly preferred. Previously, it was recognized that when magnetic beads are used in the quantitative single-molecule detection of proteins, there is a problem that the magnetic beads are suspended in the solution and it is difficult for quantitative detection; and there is a difference in brightness during the imaging process due to the directionality of the magnetic beads. However, the inventors of the present application surprisingly find that where single-molecule quantitative detection of proteins is intended to be performed, using magnetic beads as the carrier could achieve a particularly excellent sensitivity and increase significantly the detection efficiency, when compared with using a glass chip as the carrier.

The surface of the solid-phase carrier is modified with an active functional group capable of covalent coupling with antibodies, for example, one or more of hydroxyl, carboxyl, amino, succinimidyl ester, sulfonyl (such as tosyl) and groups derived therefrom.

The capture antibody could be, according to the specificity of antibody, either one or both of a polyclonal antibody and a monoclonal antibody. The capture antibody could be, according to the source, one or more of a murine antibody, a rabbit antibody, a goat antibody, and an alpaca antibody. Specific examples include Hytest 19C7, Hytest 20C6, Hytest16A11, Medix 2703, Meridian M86101M, Biospacific A45160, and Biospacific G-131-C.

The detection antibody could be, according to the specificity of antibody, either one or both of a polyclonal antibody and a monoclonal antibody. The detection antibody could be, according to the source, one or more of a murine antibody, a rabbit antibody, a goat antibody, and an alpaca antibody. Specific examples include Hytest 16A11, Medix 2704, Meridian M86201M, and Biospacific A45502.

The anti-detection antibody could be, according to the source of detection antibody, one or more of an anti-mouse antibody, an anti-rabbit antibody, an anti-goat antibody, and an anti-alpaca antibody. The anti-detection antibody could be, according to the source, one or more of a murine secondary antibody, a rabbit secondary antibody, a goat secondary antibody, and an alpaca secondary antibody.

The in-situ signal enhancing nanoparticles refer to a material, of which the fluorescent signal is enhanced in situ to a level that can be detected by a conventional optical imaging device, comprises a luminescent material and a nanoparticle carrier.

In the in-situ signal enhancing nanoparticles, the nanoparticle carrier plays a very important role. For example, it can be bound with more luminescent material to make the luminescent signal stronger; provides sites for functional modification; can bind a large number of antibodies, to improve the reactivity; and provides the possibility to realize the single-molecule detection with a conventional fluorescence microscope. If there is no nanoparticle carrier, the single-molecule detection cannot be achieved. The nanoparticle carrier could be, according to materials, one or more of silica, polystyrene, polyacrylamide, polymethyl (meth) acrylate, dextran, agarose, and inorganic metal compounds. The nanoparticle carrier includes, according to structure, one or more of a hollow structure, a core-shell structure, a perforated structure, an alloy structure, and a hydrogel structure. Among them, the nanoparticle carrier is preferably silica, polyacrylamide, polystyrene, and dextran, and particularly preferably polyacrylamide, from the perspective of uniformly distributing the luminescent material and increasing the brightness of the luminescent material.

The luminescent material in the in-situ signal enhancing nanoparticles is also necessary to realize single-molecule detection, and the sensitivity is extremely low when only a nanoparticle carrier is present. The luminescent material may be one or more of a fluorescent dye molecule, a rare earth element, a rare earth chelate, a fluorescent protein, a quantum dot, and an upconversion nanoparticle. The luminescent material is preferably fluorescein (such as fluorescein isothiocyanate), rhodamine (such as rhodamine green, rhodamine B, etc.), coumarins, quantum dots (such as CdS, CdSe, CdTe, ZnSe), rare earth elements (such as Eu, Ce) and their complexes. The luminescent material is adsorbed or wrapped on the surface or into the interior of the nanoparticle carrier by means of one or more of covalent modification, chelation, spatial wrapping, hydrophobic interaction, and electrostatic adsorption. It should be noted that, from the perspective of facilitating recognition by optical imaging and improving sensitivity, it is preferable that the luminescent material is uniformly wrapped into the interior of the nanoparticle carrier.

In the present application, the in-situ signal enhancing nanoparticles are preferably fluorescent particles formed of silica wrapped fluorescent dye molecules (such as fluorescein), fluorescent particles formed of polyacrylamide wrapped fluorescent dye molecules (such as fluorescein), fluorescent particles formed of polystyrene wrapped quantum dots, fluorescent particles formed of polystyrene wrapped rare earth elements or rare earth chelates, fluorescent particles formed of dextran wrapped fluorescent protein, and fluorescent particles formed of cross-linked agarose wrapped quantum dots.

It should be noted that the above-mentioned luminescent material and nanoparticle carrier can be combined in various manners.

In the present application, the surface of the in-situ signal enhancing nanoparticles is modified with an active functional group capable of covalent coupling with antibodies, DNA or RNA, including one or more of hydroxyl, carboxyl, amino, mercapto, alkenyl, alkynyl, succinimidyl ester and groups derived therefrom.

In the present application, the surface of the in-situ signal enhancing nanoparticles may be modified with a linking arm of a certain length. The linking arm includes a multi-carbon straight chain, a multi-carbon branched chain, a polymer chain, a peptide chain, a protein, and a nucleic acid molecule. The length of the linking arm is preferably 1-100 nm, more preferably 2-20 nm and most preferably 5-10 nm.

In the present application, the particle size of the in-situ signal enhancing nanoparticles needs to be strictly controlled within the range of 180-350 nm, for example, 190 nm, 200 nm, 210 nm, 220 nm, 230 nm, 240 nm, 250 nm, 260 nm, 270 nm, 280 nm, 290 nm, 300 nm, 310 nm, 320 nm, 330 nm, 340 nm, 350 nm. The inventors of the present application find that if the particle size of the in-situ signal enhancing nanoparticles is less than 180 nm, for example, 150 nm, no signal can be detected by a conventional optical imaging device. If the particle size is greater than 480 nm, for example, 500 nm, the detection sensitivity is very low, and it is difficult to achieve the sensitivity required in clinic. The particle size of the in-situ signal enhancing nanoparticles is preferably 200-350 nm, more preferably 220-350 nm, more preferably over 220 nm and less than 330 nm, and most preferably over 220 nm and less than 300 nm. It should be noted that the particle size may be a primary particle size or a secondary particle size. The secondary particle size refers to a particle size formed after the agglomeration of first particles and second particles.

An appropriate particle size of the in-situ signal enhancing nanoparticles can be obtained by adjusting the weight ratio of the fluorescent material to the nanoparticle carrier, the type of the nanoparticle carrier, the type of the fluorescent material, the type and amount of solvent, and other parameters.

In Step (1) of the present application, the incubation temperature of the sample and the capture antibody is 10 - 50°C, preferably 20 - 40°C, and particularly preferably 37°C; and the incubation time is 1-60 min, preferably 3 -30 min, and particularly preferably 10 - 30 min. In Step (2) of the present application, the incubation temperature of the detection antibody (or in-situ signal enhancing nanoparticles bounded with detection antibody) and the sample is 10 - 50°C, preferably 20 - 40°C, and particularly preferably 37°C; and the incubation time is 1 - 60 min, preferably 2 - 30 min, and particularly preferably 15 - 25 min.

In the present application, the optical imaging device mainly includes an excitation light source, an objective lens, an optical filter, a light sensing element, a data acquisition module, a data processing module, and a dichroic mirror (where an upright metallurgical microscope is used, the dichroic mirror may be omitted). The excitation light source is an optical emitting device used for exciting an optical signal from the sample after reaction. The objective lens is used for acquisition and amplification of a signal from the sample to be tested. The dichroic mirror is used for reflection of the excitation light path and collection of the optical signal from the sample. The optical filter is used for filtering the excitation light band and filtering the emission light signal from the sample. The light sensing element is used for collecting the optical signal from the sample. The data acquisition module is configured to receive the optical signal captured by the light sensing element and convert it into a digital signal. The data processing module is configured for conversion of the digital signal and formation and processing of an optical image.

In some examples of the device, the excitation light source includes one or more of a gas laser, a solid laser, a semiconductor laser, a liquid laser and a free electron laser. In some examples of the device, the objective lens includes, according to the magnification, one or more of 1X, 2X, 4X, 5X, 10X, 20X, 40X, 50X and 100X. The objective lens includes, according to field curvature correction, plane objective lens or curved objective lens. In some examples of the device, the light sensing element includes one or two of charge coupled device (CCD) or complementary metal-oxide semiconductor (CMOS).

As mentioned above, by adopting a specific detection system, the present application imposes a low requirement on the optical imaging device, and a conventional optical imaging device (i.e., an optical imaging device that does not break through the optical diffraction limit) can be used, with no need of expensive imaging devices that break through the optical diffraction limit, such as total internal reflection fluorescence microscope, epifluorescence microscope, scanning near-field optical microscope, and confocal fluorescence microscope.

In the present application, the target molecule concentration could be calculated by two ways: the single molecule counting pattern and the fluorescence intensity integration pattern. In the single molecule counting pattern, the number of bright spots formed by the in-situ signal enhancing nanoparticles in the generated image is directly analyzed and calculated, and then directly or indirectly converted into the concentration information of the target molecule in the sample. The expression "directly converted to the concentration information of target molecule in the sample" refers to absolute quantification, that is, conversion into the concentration information without calibration with a standard curve. The expression "indirectly converted to the concentration information of target molecule in the sample" refers to conversion into the concentration information by using the number of bright spots and a standard curve. In the fluorescence intensity integration pattern, the area of bright spots formed by the in-situ signal enhancing nanoparticles in the generated image is calculated and integrated, and then the integrated result is divided by a specific parameter, for example, average bright spot area formed by each in-situ signal nanoparticle in average or bright spot area related variables (such as power, square root, polynomial, etc.). Then, the approximate number of in-situ signal enhancing nanoparticles is obtained by conversion, and then converted into the concentration information of the target molecule in the sample. The average bright spot area is obtained by calculating and averaging the bright spot areas of single molecules at a low concentration. In order to obtain a larger dynamic detection range, it is important to use single molecule counting pattern in the low concentration range and fluorescence intensity integration pattern in the high concentration range, and then the standard curves drawn in these two patterns are combined to draw a complete standard curve. It should be noted that the boundary between the low concentration and high concentration is generally the concentration when more than one molecule to be detected is bound on the surface of a magnetic bead, or can be preferably the concentration when 0.5 molecule to be detected or 2 molecules to be detected is/are bound on the surface of a magnetic bead on average according to the result of standard curve fitting.

### <Second Embodiment>

A second embodiment of the present application is shown below.

A quantitative single-molecule detection and analysis method includes the following steps:
(1) allowing a detection antibody to bind to a second site of a target molecule in a sample, and then adding in-situ signal enhancing nanoparticles able to directly or indirectly bind to the detection antibody; or
   allowing the in-situ signal enhancing nanoparticles to bind to the detection antibody to form a complex material first, and then adding the complex material to the sample to allow the complex material to bind to the second site of the target molecule in the sample;
   wherein the in-situ signal enhancing nanoparticles comprise a luminescent material and a nanoparticle carrier, and have a particle size of 180-350 nm, and the luminescent material is a fluorescent material;
(2) immobilizing a capture antibody able to bind to the target molecule to a solid-phase carrier, and then allowing the capture antibody to bind to a first site of the target molecule to capture the target molecule;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation,
   wherein the solid-phase carrier comprises magnetic beads.

The second embodiment differs from the first embodiment only in the order of implementation of the steps, and other conditions such as in-situ signal enhancing nanoparticles are the same.

### <Third Embodiment>

A third embodiment of the present application is shown below.

A quantitative single-molecule detection and analysis method comprises the following steps:
(1) immobilizing a capture probe able to bind to a target molecule to a solid-phase carrier, wherein the capture probe is complementary to a first sequence of the target molecule; and capturing the target molecule in a sample by the capture probe;
(2) adding a detection probe that is complementary to a second sequence of the target molecule, to form a three-strand hybrid structure of capture probe-target molecule-detection probe, and then adding in-situ signal enhancing nanoparticles able to directly or indirectly bind to the detection probe; or
   allowing the detection probe to bind to the in-situ signal enhancing nanoparticles to form a complex material, and then adding the complex material;
   wherein the in-situ signal enhancing nanoparticles comprise a luminescent material and a nanoparticle carrier, and have a particle size of 180-350 nm, and the luminescent material is a fluorescent material;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation , wherein the solid-phase carrier comprises magnetic beads.

In this embodiment, the target molecule includes DNA or RNA.

The detection reagent includes the solid-phase carrier, the capture probe, the detection probe and the in-situ signal enhancing nanoparticles. The solid-phase carrier is used for the separation and cleaning of the test sample and the reagent. The capture probe is immobilized on the surface of the solid-phase carrier by chemical modification, and can be hybridized and bound to a part of the molecules to be detected to separate it from the sample. The detection probe can be hybridized and bound to another part of the molecules to be detected, and the end of the detection probe far away from the hybridization position is chemically covalently bonded to the in-situ signal enhancing nanoparticles. The in-situ signal enhancing nanoparticles can emit a sufficiently strong optical signal to form an independently distinguishable image signal on the optical imaging device.

The solid-phase carrier includes, according to the morphology, one or more of magnetic beads, perforated plates, centrifuge tubes, chips, micro-scale microspheres and nano-scale microspheres; includes, according to the material, one or more of organic polymers, silica, and silicon; and includes, according to the function, one of magnetic solid-phase carrier and non-magnetic solid-phase carrier. Among these solid-phase carriers, magnetic beads are particularly preferred. Previously, it was recognized that when magnetic beads are used in the quantitative detection of nucleic acid, there is a problem that the magnetic beads are suspended and it is difficult for quantitative detection; and there is a difference in brightness due to the directionality of the magnetic beads. The inventors of the present application find that where single-molecule quantitative detection of nucleic acids is intended to be performed, using magnetic beads as the carrier could achieve a particularly excellent sensitivity and increase significantly the detection efficiency, when compared with using a glass chip as the carrier.

In the present application, the surface of the solid-phase carrier is modified with an active functional group capable of covalent coupling with a probe, including one or more of hydroxyl, carboxyl, amino, mercapto, alkenyl, alkynyl, succinimidyl ester and groups derived therefrom.

In the present application, the capture probe may be ribonucleic acid or deoxyribonucleic acid, having a sequence that is complementary to a sequence of the molecules to be detected to form double-strand hybridization.

In the present application, one end of the capture probe is modified with one or more of carboxyl, amino, mercapto and succinimidyl ester, which can be covalently bonded to the surface of the solid-phase carrier, thereby stably binding to the surface of the solid-phase carrier.

In the present application, the detection probe may be ribonucleic acid or deoxyribonucleic acid, having a sequence that is complementary to another sequence (different from the sequence complementary to the capture probe) of the molecules to be detected to form double-strand hybridization. As such, a three-strand hybrid structure of capture probe-target molecule-detection probe is formed.

In the present application, one end of the detection probe is modified with one or more of carboxyl, amino, mercapto and succinimidyl ester, which can be covalently coupled to the in-situ signal enhancing nanoparticles, thereby stably binding to the surface of the in-situ signal enhancing nanoparticles.

Other conditions, such as in-situ signal enhancing nanoparticles, optical imaging device, and calculation method of target molecule concentration, are the same as those in the first embodiment described above.

### <Fourth Embodiment>

A fourth embodiment of the present application is shown below.

A quantitative single-molecule detection and analysis method comprises the following steps:
(1) allowing a detection probe to be complementary to a second sequence of a target molecule in a sample, and then adding in-situ signal enhancing nanoparticles directly or indirectly binding to the detection probe; or
   allowing the in-situ signal enhancing nanoparticles to bind to the detection probe to form a complex material in advance, and then allowing the complex material to be complementary to a second sequence of the target molecule in the sample;
   wherein the in-situ signal enhancing nanoparticles comprise a luminescent material and a nanoparticle carrier, and have a particle size of 180-350 nm, and the luminescent material is a fluorescent material;
(2) immobilizing a capture probe able to bind to the target molecule to a solid-phase carrier, and then allowing the capture antibody to be complementary to the first sequence of the target molecule to capture the target molecule;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation, wherein the solid-phase carrier comprises magnetic beads.

The fourth embodiment differs from the third embodiment only in the order of implementation of the steps, and other conditions such as in-situ signal enhancing nanoparticles are the same.

### <Fifth Embodiment>

A fifth embodiment of the present application is shown below.

A quantitative single-molecule detection system comprises:
(1) a detection reagent, comprising:
   (a) a capture antibody able to bind to a first site of a target molecule to capture the target molecule in a sample; (b) a detection antibody able to bind to a second site of the target molecule and able to bind to in-situ signal enhancing nanoparticles; and (c) the in-situ signal enhancing nanoparticles comprising a luminescent material and a nanoparticle carrier and having a particle size of 180-350 nm, and the luminescent material is a fluorescent material; (d) a solid-phase carrier, the solid-phase carrier comprises magnetic beads and is able to immobilize the capture probe; or
   (a) a capture probe able to bind to a first sequence of a target molecule to capture the target molecule in a sample; (b) a detection probe able to bind to a second sequence of the target molecule to form a three-strand hybrid structure of capture probe-target molecule-detection probe; and (c) in-situ signal enhancing nanoparticles able to bind to the detection probe, comprising a luminescent material and a nanoparticle carrier and having a particle size of 180-350 nm, and the luminescent material is a fluorescent material; (d) a solid-phase carrier, the solid-phase carrier comprises magnetic beads and is able to immobilize the capture probe; and
(2) an optical imaging device including an excitation light source and an optical signal acquisition unit.

The detection system of the present application includes the detection reagent and the optical imaging device. The detection reagent comprises the capture antibody, the detection antibody and particular in-situ signal enhancing nanoparticles (when used in the detection of proteins, polysaccharides or biologically active small molecules), or comprises the capture probe, the detection probe and particular in-situ signal enhancing nanoparticles (when used in the detection of DNA or RNA). The optical imaging device is a conventional optical imaging device, with no need of expensive optical imaging devices that break through the optical diffraction limit, such as total internal reflection fluorescence microscope, epifluorescence microscope, scanning near-field optical microscope, and confocal fluorescence microscope.

### [Examples]

Hereinafter, the present application will be described in further detail by way of examples and comparative examples; however, the present application is not limited thereto. The scope of protection is limited by the appended claims.

### 1. Determination of particle size of the in-situ signal enhancing nanoparticles

Fluorescent silica nanoparticles are used as an example. The fluorescent silica nanoparticles obtained in each example and comparative example were 1000-fold diluted with water, and then 100 µL was dripped on the surface of a clean silicon chip, and dried. 5 nm-thick platinum was sputtered and deposited on the surface by using a small sputtering instrument. After imaging and analysis by SEM (SU3900 manufactured by Hitachi High-tech Corporation, Japan), the particle size was calculated.

Fluorescent polyacrylamide nanoparticles are used as an example. The obtained fluorescent polyacrylamide nanoparticles are 1000-fold diluted with pure water, and the particle size is measured by Marvin particle size analyzer (Zetasizer Nano S90).

### 2. Single molecule imaging

A conventional fluorescence microscope, such as Nikon Eclipse Ti-U fluorescence microscope, is used to perform single molecule imaging. In addition, other fluorescence microscopes of Nikon Eclipse Ti series and Leica DMi8 fluorescence microscope can also be used.

### 3. Plotting of standard curve

In the present application, the combined use of the single molecule counting pattern and the fluorescence intensity integration pattern can significantly increase the dynamic detection range of the standard curve of the detection marker. The specific embodiment is as follows:
When the concentration of the molecules to be detected is low, the number of magnetic beads is more than the number of molecules to be detected binding to the magnetic beads, and thus the single molecule counting pattern is used to plot standard curves for various concentrations of samples of the molecules to be detected.

When the concentration of the molecules to be detected exceeds a certain threshold, more than one molecules to be detected may be bound to the surface of one magnetic bead, and single-molecule signals tend to superimpose, resulting in deviations in the detection results. Therefore, fluorescence intensity integration pattern is more suitable.

Specifically, when the number of single molecules in an image does not exceed the set threshold, the single molecule counting pattern is used to plot a standard curve. When the number of single molecules in an image exceeds the set threshold, the fluorescence intensity integration pattern is used, wherein the total fluorescence intensity area is divided by the average fluorescence intensity area of each molecule to convert to "approximate number of single molecules" for plotting a standard curve.

Finally, the standard curve obtained by the single molecule counting pattern and the standard curve obtained by the fluorescence intensity integration pattern are combined, and the curves are fitted by using a fitting formula to plot a complete standard curve.

### Example 1: Detection of cTnI antigen molecules in human serum by magnetic bead method (fluorescent silica nanoparticles with a particle size of 220 nm).

### 1. Reagents used in experiment

Tosyl activated M280 magnetic beads (Thermo), capture antibody (Hytest 19C7), detection antibody (Hytest 16A11), silane coupling agent (APTES), aqueous ammonia, ethyl orthosilicate (TEOS), fluorescein isothiocyanate (FITC), succinic anhydride, serum samples to be tested, PBS buffer, Buffer C (3 mM (NH₄)₂SO₄ dissolved in 10 mM PBS buffer, pH=7.4), Buffer D (0.01% NaCl, 0.5% BSA dissolved in 10 mM PBS, pH=7.4), Buffer E (0.0088% NaCl, 0.1% BSA dissolved in 10 mM PBS, pH=7.4), N-hydroxysuccinimide (NHS), 1-(3-dimethylaminopropyl)-3-ethylcarbodiimide hydrochloride (EDC), microsphere preservation solution, sample diluent and PBS washing solution.

### 2. Preparation method

### 2.1. Synthesis of fluorescent silica nanoparticles and surface modification by carboxylation

(1) 16.5 mL of absolute ethanol, 1.2 mL of aqueous ammonia, 0.9 mL of ultrapure water, 0.7 mL of TEOS, 0.3 µL of APTES, and 0.6 mg of FITC were added to a flat-bottom plastic synthetic bottle, mixed well, and stirred at 400 rpm for 5 hrs at 50°C. After washing with 20 mL of absolute ethanol (5x) and centrifugation, silica microspheres having FITC wrapped therein were obtained and suspended in 20 mL of absolute ethanol.
(2) Equimolar APTES and succinic anhydride were dissolved in 1 mL of DMF, stirred at room temperature for 3 hrs, and then added to the absolute ethanol solution with suspending silica microspheres (having FITC uniformly distributed therein) obtained in (1). 2 mL of ultrapure water and 1.2 mL of aqueous ammonia were added, and stirred at 400 rpm for 5 hrs at 50°C. The resulting product was washed with 20 mL of absolute ethanol (3x) and centrifuged, followed by washing with 20 mL of ultrapure water (3x) and centrifugation, and then was re-suspended in 20 mL of ultrapure water to obtain fluorescent silica nanoparticles carboxylated on the surface and having a particle size of 220 nm.

### 2.2. Covalent coupling of magnetic beads with capture antibody

(1) 166.6 µL of 30 mg/mL tosyl-activated M280 magnetic beads were washed with 10 mM PBS buffer (5x), and then the buffer was removed.
(2) 100 µg of the capture antibody (Hytest 19C7) was diluted with 150 µL of 10 mM PBS buffer, added to the magnetic beads obtained in (1), and mixed evenly. 100 µL of Buffer C was added, rotated and mixed uniformly at 37°C, and reacted for 1 hr.
(3) It was washed 5 times with 10 mM PBS buffer, and then 1 mL of Buffer D was added for blocking. It was rotated and mixed uniformly, and reacted for 1 hr at 37°C.
(4) It was washed 5 times with 10 mM PBS buffer, and preserved in 250 µL of Buffer E for later use.

### 2.3. Covalent coupling of detection antibody and fluorescent silica nanoparticles

(1) 10 µL of fluorescent silica nanoparticles were added to 40 µL of PBS buffer and ultrasonicated for 1 min.
(2) 0.005 g of EDC was dissolved in 50 µL of PBS buffer, and 0.0135 g of NHS was dissolved in 150 µL of PBS buffer.
(3) The fluorescent silica nanoparticles were centrifuged at 12000 rpm, and the supernatant was removed. The fluorescent silica nanoparticles were re-suspended in 50 µL of PBS buffer, ultrasonicated for 1 min, added with 2.5 µL of EDC solution, and ultrasonicated for 1 min. Then, 7.5 µL of NHS solution was added, mixed well, and reacted at 37°C for 15 min with rotation and mixing. It was centrifuged at 12000 rpm for 15 min, the supernatant was removed, and the nanoparticles were re-suspended in 50 µL of PBS buffer.
(4) 20 µg of detection antibody (Hytest 16A11) was added, and reacted at 37°C for 2 hrs with rotation and mixing.
(5) 25 µL of Buffer D was added for blocking at 37°C for 1 hr. Then, it was centrifuged at 12000 rpm for 15 min and then re-suspended and preserved in 100 µL of Buffer E.

### 3. Experimental method

### Plotting of standard curve

(1) The cTnI antigen was diluted with fetal calf serum to give a concentration of 0, 0.01, 0.1, 0.5, 1, 5, 10 and 100 pg/mL.
(2) The magnetic beads labeled with the capture antibody were diluted to 1 mg/mL. 50 µL was taken and added to 50 µL of the samples of various concentration obtained in (1), and incubated at 37°C for 60 min. It was washed three times with 100 µL of washing buffer to remove the residual sample, and the supernatant was aspirated off.
(3) 10 µL of fluorescent silica nanoparticles bounded with the detection antibody was added, incubated at 37°C for 45 min, and washed with the washing buffer (4x) to remove the remaining fluorescent silica nanoparticles. The supernatant was removed.
(4) 5 µL of the detection solution was added to re-suspend the magnetic beads, and transferred to the detection well. The magnetic beads were attracted by a magnet to the bottom of the detection well, and subjected to single molecule imaging by using a fluorescence microscope (Nikon Eclipse Ti-U). Subsequent statistical analysis of the number of single molecules was performed by single molecule counting pattern in combination with fluorescence intensity integration pattern.
(5) The detection of a series of concentrations was conducted. 6 replicates were set for each concentration point. A standard curve was plotted from the test results, and the CV% for each point was calculated.

### 4. Experimental results

### Results from standard curve

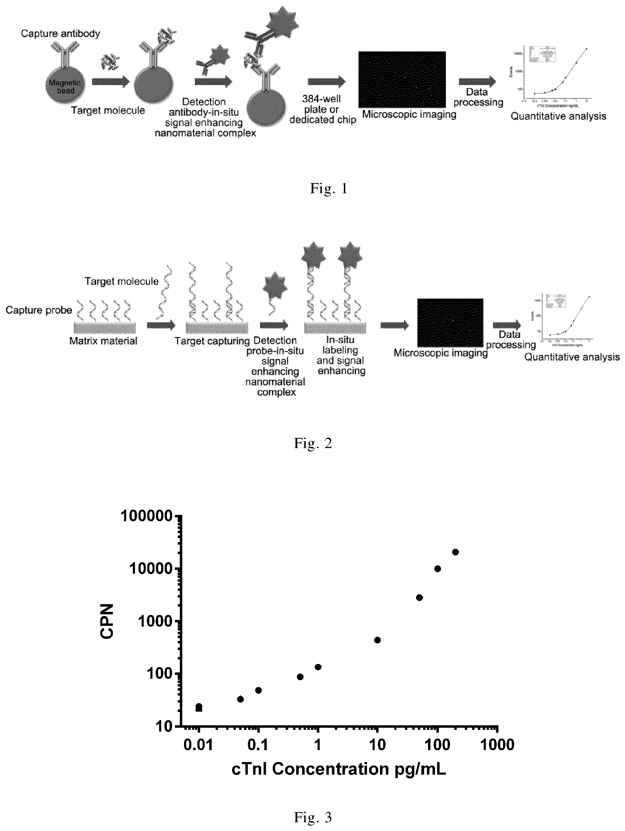

The detection results are shown in Fig. 3. It can be seen that in this example, the detection range of cTnI is 30 fg/mL - 10 ng/mL. In this range, the number of single molecule signals (i.e., CPN) is in a good linear relationship with the sample concentration. The lower detection limit can reach 30 fg/mL.

### Comparative Example 1: Detection of cTnI antigen molecules in human serum by magnetic bead method (fluorescent silica nanoparticles with a particle size of 150 nm)

### 1. Preparation method

### 1.1. Synthesis of fluorescent silica nanoparticles and surface modification by carboxylation

(1) 16.5 mL of absolute ethanol, 1.2 mL of aqueous ammonia, 0.9 mL of ultrapure water, 0.5 mL of TEOS, 0.3 µL of APTES, and 0.6 mg of FITC were added to a flat-bottom plastic synthetic bottle, mixed well, and stirred at 400 rpm for 5 hrs at 50°C. After washing with 20 mL of absolute ethanol (5x) and centrifugation, silica microspheres having FITC wrapped therein were obtained and suspended in 20 mL of absolute ethanol.
(2) Equimolar APTES and succinic anhydride were dissolved in 1 mL of DMF, stirred at room temperature for 3 hrs, and then added to the absolute ethanol solution with suspending silica microspheres obtained in (1). 2 mL of ultrapure water and 1.2 mL of aqueous ammonia were added, and stirred at 400 rpm for 5 hrs at 50°C. The resulting product was washed with 20 mL of absolute ethanol (3x) and centrifuged, followed by washing with 20 mL of ultrapure water (3x) and centrifugation, and then was re-suspended in 20 mL of ultrapure water to obtain fluorescent silica nanoparticles carboxylated on the surface and having a particle size of 150 nm.

### 1.2. Covalent coupling of magnetic beads with capture antibody

The operation steps were the same as those in Example 1.

### 1.3. Covalent coupling of detection antibody and fluorescent silica nanoparticles

The operation steps were the same as those in Example 1, except that fluorescent silica nanoparticles carboxylated on the surface and having a particle size of 150 nm were used.

### 2. Experimental method

### Plotting of standard curve

The operation steps were the same as those in Example 1.

### 3. Experimental results

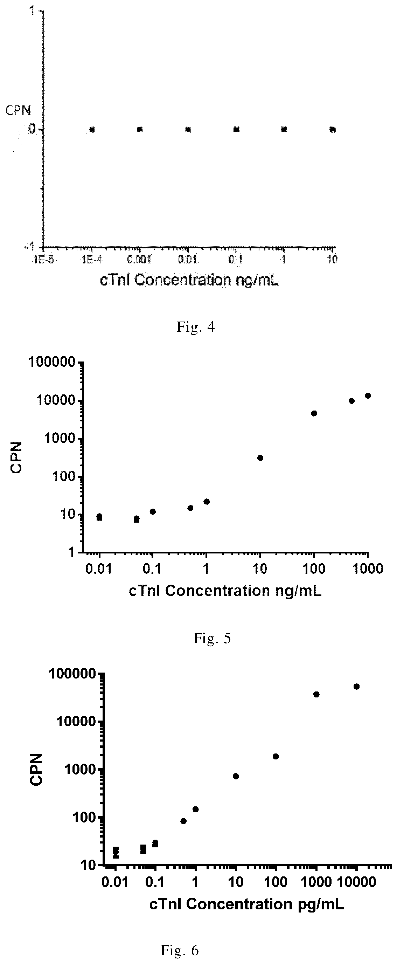

It can be seen from Fig. 4 that when fluorescent silica nanoparticles with a particle size of 150 nm were used, no fluorescence signal could be detected at all by the detection device. It can be seen that fluorescent silica nanoparticles of this size cannot be used for quantitative single-molecule detection. Since the reagent is completely unresponsive to the sample, no clinical sample was tested.

### Comparative Example 2: Detection of cTnI antigen molecules in human serum by magnetic bead method (fluorescent silica nanoparticles with a particle size of 500 nm)

### 1. Preparation method

### 1.1. Synthesis of fluorescent silica nanoparticles and surface modification by carboxylation

(1) 16.5 mL of absolute ethanol, 1.0 mL of aqueous ammonia, 0.9 mL of ultrapure water, 1.2 mL of TEOS, 0.3 µL of APTES, and 0.6 mg of FITC were added to a flat-bottom plastic synthetic bottle, mixed well, and stirred at 400 rpm for 5 hrs at 50°C. was After washing with 20 mL of absolute ethanol (5x) and centrifugation, silica microspheres having FITC wrapped therein were obtained and suspended in 20 mL of absolute ethanol.
(2) Equimolar APTES and succinic anhydride were dissolved in 1 mL of DMF, stirred at room temperature for 3 hrs, and then added to the absolute ethanol solution with suspending silica microspheres obtained in (1). 2 mL of ultrapure water and 1.2 mL of aqueous ammonia were added, and stirred at 400 rpm for 5 hrs at 50°C. The resulting product was washed with 20 mL of absolute ethanol (3x) and centrifuged, followed by washing with 20 mL of ultrapure water (3x) and centrifugation, and then was re-suspended in 20 mL of ultrapure water to obtain fluorescent silica nanoparticles carboxylated on the surface and having a particle size of 500 nm.

### 1.2. Covalent coupling of magnetic beads - capture antibody

The operation steps were the same as those in Example 1.

### 1.3. Covalent coupling of detection antibody and fluorescent silica nanoparticles

The operation steps were the same as those in Example 1, except that fluorescent silica nanoparticles carboxylated on the surface and having a particle size of 500 nm were used.

### 2. Experimental method

### Plotting of standard curve

The operation steps were the same as those in Example 1.

### 3. Experimental results

The detection result is shown in Fig. 5. It can be seen that in Comparative Example 2, the lower detection limit of cTnI is only 0.1 ng/mL (that is, 100 pg/mL), and the sensitivity is poor. In Comparative Example 2, the use of particles with a particle size of 500 nm results in a much lower detection sensitivity than that of Example 1.

### Example 2: Detection of cTnI antigen molecules in human serum by magnetic bead method (fluorescent silica nanoparticles with a particle size of 350 nm).

### 1. Preparation method

### 1.1. Synthesis of fluorescent silica nanoparticles and surface modification by carboxylation

(1) 16.5 mL of absolute ethanol, 1.0 mL of aqueous ammonia, 0.9 mL of ultrapure water, 0.7 mL of TEOS, 0.3 µL of APTES, and 0.6 mg of FITC were added to a flat-bottom plastic synthetic bottle, mixed well, and stirred at 400 rpm for 5 hrs at 50°C. was After washing with 20 mL of absolute ethanol (5x) and centrifugation, silica microspheres having FITC wrapped therein were obtained and suspended in 20 mL of absolute ethanol.
(2) Equimolar APTES and succinic anhydride were dissolved in 1 mL of DMF, stirred at room temperature for 3 hrs, and then added to the absolute ethanol solution with suspending silica microspheres obtained in (1). 2 mL of ultrapure water and 1.2 mL of aqueous ammonia were added, and stirred at 400 rpm for 5 hrs at 50°C. The resulting product was washed with 20 mL of absolute ethanol (3x) and centrifuged, followed by washing with 20 mL of ultrapure water (3x) and centrifugation, and then was re-suspended in 20 mL of ultrapure water to obtain fluorescent silica nanoparticles carboxylated on the surface and having a particle size of 350 nm.

### 1.2. Covalent coupling of magnetic beads with capture antibody

The operation steps were the same as those in Example 1.

### 1.3. Covalent coupling of detection antibody - fluorescent silica nanoparticles

The operation steps were the same as those in Example 1, except that fluorescent silica nanoparticles carboxylated on the surface and having a particle size of 350 nm were used.

### 2. Experimental method

### Plotting of standard curve

The operation steps were the same as those in Example 1.

### 3. Experimental results

The detection result is shown in Fig. 6. It can be seen that in Comparative Example 2, the lower detection limit of cTnI is 80 fg/mL, and the sensitivity is excellent.

### Comparative Example 3

The operation steps were the same as those in Example 1, except that fluorescent silica nanoparticles carboxylated on the surface and having a particle size of 160 nm were used. The result of the lower detection limit is the same as that in Comparative Example 1.

### Example 3

The operation steps were the same as those in Example 1, except that fluorescent silica nanoparticles carboxylated on the surface and having a particle size of 180 nm were used. The results are shown in Table 1.

### Example 4

The operation steps were the same as those in Example 1, except that fluorescent silica nanoparticles carboxylated on the surface and having a particle size of 250 nm were used. The results are shown in Table 1.

### Example 5

The operation steps were the same as those in Example 1, except that fluorescent silica nanoparticles carboxylated on the surface and having a particle size of 295 nm were used. The results are shown in Table 1.

### Example 6

The operation steps were the same as those in Example 1, except that fluorescent silica nanoparticles carboxylated on the surface and having a particle size of 300 nm were used. The results are shown in Table 1.

### Example 7

The operation steps were the same as those in Example 1, except that fluorescent silica nanoparticles carboxylated on the surface and having a particle size of 400 nm were used. The results are shown in Table 1.

### Example 8

The operation steps were the same as those in Example 1, except that fluorescent silica nanoparticles carboxylated on the surface and having a particle size of 450 nm were used. The results are shown in Table 1.

### Example 9

The operation steps were the same as those in Example 1, except that fluorescent silica nanoparticles carboxylated on the surface and having a particle size of 480 nm were used. The results are shown in Table 1.

### Example 10

The operation steps were the same as those in Example 1, except that in the step of covalent coupling of the solid-phase carrier and the capture antibody, a tosyl-activated glass chip was used to replace the tosyl-activated magnetic beads. The results are shown in Table 1.

### Example 11

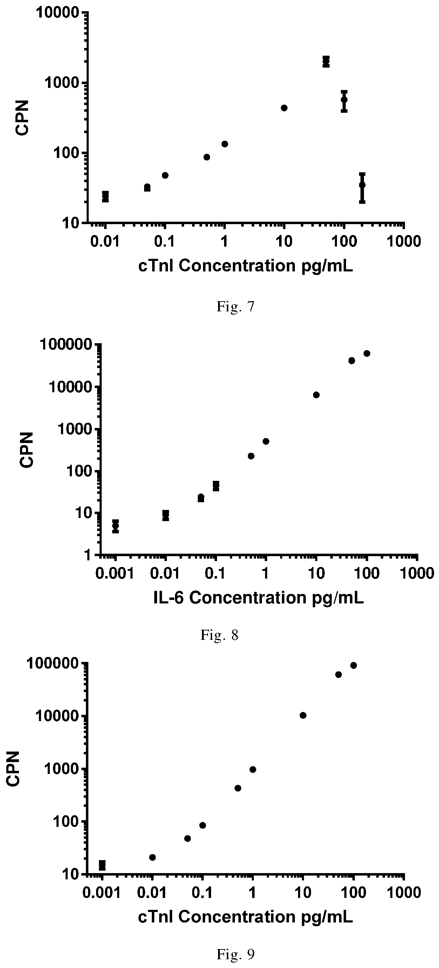

The operation steps were the same as those in Example 1, except that in the step of processing the data from single molecule imaging, the single molecule counting pattern is used in the whole concentration range to replace the combination of the single molecule counting pattern and the fluorescence intensity integration pattern. The results are shown in Fig. 7, and it can be seen that the upper detection limit is lowered and the dynamic detection range is narrowed compared to Example 1.

### Example 12

The operation steps were the same as those in Example 1, except that in the step of single-molecule imaging, the Nikon Eclipse Ti-U was replaced by a Leica DMi8 fluorescence microscope. The results are shown in Table 1.

### Example 13

The operation steps were the same as those in Example 1, except that, in Steps 2.3 and 3.1 of Example 1, instead of forming a complex of the detection antibody and the fluorescent silica nanoparticles in advance, a detection antibody modified with biotin was added to bind to a second site of the molecules to be detected, and then the fluorescent silica nanoparticles modified with streptavidin were added. The results are shown in Table 1.

### Example 14: Detection of IL-6 antigen molecules by magnetic bead method (fluorescent polyacrylamide nanoparticles with a particle size of 220 nm)

### 1. Reagents used in experiment

Tosyl-activated M280 magnetic beads (Thermo), IL-6 capture antibody (Medix 2703), IL-6 detection antibody (Medix 2704), acrylamide, N,N-methylenebisacrylamide, acrylic acid, hexane, dioctyl sulfosuccinate sodium salt, polyoxyethylene lauryl ether 35 (Brij 35), sodium bisulfite, ammonium persulfate (initiator), tetramethylethylenediamine, acrylamide-(PEG)₈-fluorescein, 1 -(3-Dimethylaminopropyl)-3-ethylcarbodiimide hydrochloride (EDC), N-hydroxysuccinimide (NHS), ethanol, serum sample to be tested, PBS buffer, Buffer C (3 mM (NH₄)₂SO₄ dissolved in 10 mM PBS buffer, pH=7.4), Buffer D (0.01% NaCl, 0.5% BSA dissolved in 10 mM PBS, pH=7.4), Buffer E (0.0088% NaCl, 0.1% BSA dissolved 10 mM PBS, pH=7.4), N-hydroxysuccinimide (NHS), 1-(3-dimethylaminopropyl)-3-ethylcarbodiimide hydrochloride (EDC), fluorescent particle preservation solution, sample diluent and PBS washing solution.

### 2. Preparation method

### 2.1. Synthesis of carboxylated fluorescent polyacrylamide nanoparticles and synthesis of its complex with detection antibody

(1) 1 mL of 10 mM PBS buffer containing 23% acrylamide, 3% N,N-methylenebisacrylamide, 2% acrylic acid, and 1% acrylamide-(PEG)₈-fluorescein was added to 20 mL of hexane. Then 2% Brij-35 was added, and stirred at 1000 rpm overnight at room temperature. 50 µL of 10% sodium sulfite solution, 24 µL of 10% ammonium persulfate and 12 µL of tetramethylethylenediamine were added. Stirring was continued for 2 hrs at room temperature. At room temperature, the hexane was completely volatilized, and the fluorescent polyacrylamide particles remaining after volatilization were re-suspended in 10 mM PBS buffer. The surfactant and residual monomer were filtered off with a 100 kD ultrafiltration tube (Millipore). The particles were re-suspended in 100 µL of 10 mM PBS buffer again.
(2) 10 µL of the fluorescent polyacrylamide nanoparticle suspension prepared in (1) was taken and diluted to 100 µL with 10 mM PBS buffer. 10 µL of an aqueous solution containing 0.5% EDC and 5 µL of an aqueous solution containing 0.5% NHS were added for activation at 37°C for 1 hr. The residual activator was removed by a 100 kD ultrafiltration tube, and 100 µL of 10 mM PBS buffer was added to re-suspend the nanoparticles. Then, 20 µg of IL-6 detection antibody (Medix 2704) was added, and reacted at 37°C for 2 hrs. The residual antibody was removed by using a 150KD ultrafiltration tube, and the nanoparticles were re-suspended in 100 µL of 10 mM PBS buffer. The ultrafiltration and resuspension steps were repeated once. The resulting complex of fluorescent polyacrylamide nanoparticles with the detection antibody was stored at 4°C for future use.

### 2.2. Covalent coupling of magnetic beads with capture antibody

(1) 166.6 µL of 30 mg/mL tosyl-activated M280 magnetic beads were washed with 10 mM PBS buffer (5x), and then the buffer was removed.
(2) 100 µg of the capture antibody (Medix 2703) was diluted with 150 µL of 10 mM PBS buffer, added to the magnetic beads obtained in (1), and mixed evenly. 100 µL of Buffer C was added, rotated and mixed uniformly, and reacted for 1 hr at 37°C.
(3) It was washed 5 times with 10 mM PBS buffer, and then 1 mL of Buffer D was added for blocking. It was rotated and mixed uniformly, and reacted for 1 hr at 37°C.
(4) It was washed 5 times with 10 mM PBS buffer, and preserved in 250 µL of Buffer E for later use.

### 3. Experimental method

### 3.1. Determination of particle size of fluorescent polyacrylamide nanoparticles

The obtained fluorescent polyacrylamide nanoparticles were 1000-fold diluted with pure water, and the particle size was measured by Marvin particle size analyzer.

### 3.2. Detection of IL-6 antigen

(1) The IL-6 antigen was diluted with fetal calf serum to give a concentration of 0, 0.01, 0.1, 0.5, 1, 5, 10, 50 and 100 pg/mL.
(2) The magnetic beads labeled with the capture antibody were diluted to 0.1 mg/mL. 50 µL was taken and added to 50 µL of the samples of various concentration obtained in (1), and incubated at 37°C for 30 min. It was washed three times with 100 µL of washing buffer to remove the residual sample, and the supernatant was aspirated off.
(3) 10 µL of fluorescent polyacrylamide nanoparticles bounded with the detection antibody was added, incubated at 37°C for 15 min, and washed with the washing buffer (4x) to remove the remaining fluorescent polyacrylamide nanoparticles. The supernatant was removed.
(4) 5 µL of the detection solution was added to re-suspend the magnetic beads, and transferred to the detection well. The magnetic beads were attracted by a magnet to the bottom of the detection well, and subjected to single molecule imaging by using a fluorescence microscope (Nikon Eclipse Ti-U). Subsequent statistical analysis of the number of single molecules was performed by fluorescence intensity integration pattern.
(5) The detection of a series of concentrations was conducted. 3 replicates were set for each concentration point. A standard curve was plotted from the test results, and the CV% for each point was calculated.

### 4. Experimental results

### 4.1. Determination of particle size of fluorescent polyacrylamide nanoparticles

The particle size of the fluorescent polyacrylamide nanoparticles is determined to be 220 nm by Marvin particle size analyzer.

### 4.2. Detection result of IL-6 antigen

The detection result of IL-6 antigen is shown in Fig. 8. It can be seen that the diluted sample with a concentration of 0.01 pg/mL can be effectively distinguished from the background. The lower detection limit in this example is calculated to be 0.006 pg/mL (that is, 6 fg/mL). It can be seen that the sensitivity of the detection method of the present application is extremely excellent.

### Example 15: Detection of cTnI antigen molecules by magnetic bead method (fluorescent polyacrylamide nanoparticles with a particle size of 220 nm)

The operation steps were the same as those in Example 14, except that the capture antibody Medix 2703 in Example 14 was replaced by Hytest 16A11 (that is, the capture antibody of cTnI antigen), the detection antibody Medix 2704 was replaced by Hytest 19C7 (that is, the detection antibody of cTnI antigen), and the molecule to be detected IL-6 antigen was replaced by cTnI antigen. The detection result of cTnI antigen is shown in Fig. 9. It can be seen that the diluted sample with a concentration of 0.01 pg/mL can be effectively distinguished from the background. The lower detection limit in this example is calculated to be 0.008 pg/mL (that is, 8 fg/mL). It can be seen that the sensitivity of the detection method of the present application is extremely excellent.

### Example 16

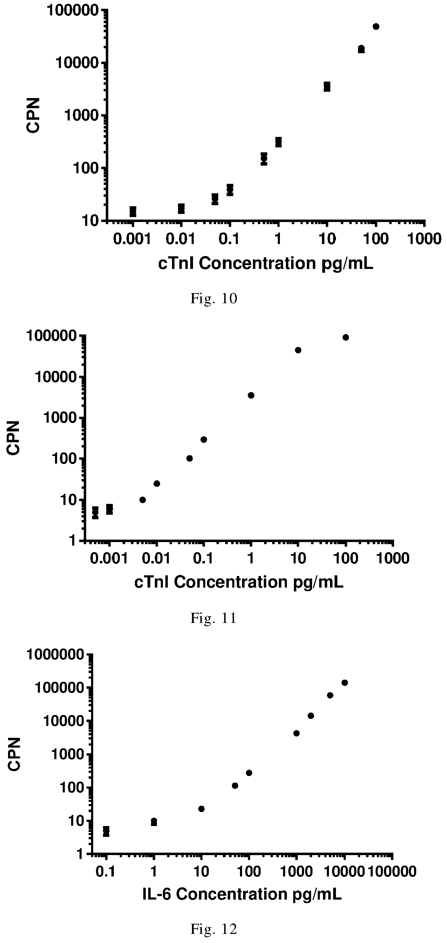

The operation steps were the same as those in Example 15, except that fluorescent polyacrylamide nanoparticles carboxylated on the surface and having a particle size of 300 nm were used. The detection result of cTnI antigen is shown in Fig. 10. It can be seen that the diluted sample with a concentration of 0.01 pg/mL can be effectively distinguished from the background. The lower detection limit in this example is calculated to be 0.020 pg/mL (that is, 20 fg/mL). It can be seen that the sensitivity of the detection method of the present application is excellent.

### Example 17: Detection of cTnI antigen molecules by magnetic bead method (fluorescent polyacrylamide nanoparticles with a secondary particle size of 250 nm)

### 1. Reagents used in experiment

Tosyl activated M280 magnetic beads (Thermo), capture antibody (Hytest 16A11), detection antibody (Hytest 19C7), streptavidin (SA, Roche), acrylamide, acrylamide-(PEG)₈-biotin and acrylamide-(PEG)₈-fluorescein.

### 2. Preparation method

### 2.1. Synthesis of first fluorescent polyacrylamide particles and modification by carboxylation

(1) 1 mL of 10 mM PBS buffer containing 23% acrylamide, 3% N,N-methylenebisacrylamide, 2% acrylic acid, 1% acrylamide-(PEG)₈-biotin and 1% acrylamide-(PEG)₈-fluorescein was added to 20 mL of hexane. Then 4% Brij-35 was added, and stirred at 1200 rpm overnight at room temperature. 50 µL of 10% sodium sulfite solution, 24 µL of 10% ammonium persulfate and 12 µL of tetramethylethylenediamine were added. Stirring was continued for 2 hrs at room temperature. At room temperature, the hexane was completely volatilized, and the fluorescent polyacrylamide particles remaining after volatilization were re-suspended in 10 mM PBS buffer. The surfactant and residual monomer were filtered off with a 100 kD ultrafiltration tube. The nanoparticles were re-suspended in 100 µL of 10 mM PBS buffer.
(2) 15 µL of the fluorescent polyacrylamide nanoparticle suspension prepared in (1) was taken and diluted to 100 µL with 10 mM PBS buffer. 10 µL of an aqueous solution containing 0.5% EDC and 5 µL of an aqueous solution containing 0.5% NHS were added for activation at 37°C for 1 hr. The residual activator was removed by a 100 kD ultrafiltration tube, and 100 µL of 10 mM PBS buffer was added to re-suspend the nanoparticles. Then, 20 µg of the detection antibody was added, and reacted at 37°C for 2 hrs. The residual antibody was removed by using a 150KD ultrafiltration tube, and the nanoparticles were re-suspended in 100 µL of 10 mM PBS buffer. The ultrafiltration and resuspension steps were repeated once. The resulting first polyacrylamide nanoparticle suspension was stored at 4°C for future use.

### 2.2. Synthesis of second fluorescent polyacrylamide nanoparticles and modification by carboxylation

(1) 1 mL of 10 mM PBS buffer containing 23% acrylamide, 3% N,N-methylenebisacrylamide, 2% acrylic acid, and 1% acrylamide-(PEG)₈-fluorescein was added to 20 mL of hexane. 4% Brij-35 was added, and stirred at 1200 rpm overnight at room temperature. 50 µL of 10% sodium sulfite solution, 24 µL of 10% ammonium persulfate and 12 µL of tetramethylethylenediamine were added. Stirring was continued for 2 hrs at room temperature. At room temperature, the hexane was completely volatilized, and the fluorescent polyacrylamide particles remaining after volatilization were re-suspended in 10 mM PBS buffer. The surfactant and residual monomer were filtered off by a 100 kD ultrafiltration tube. The nanoparticles were re-suspended in 100 µL of 10 mM PBS buffer.
(2) 15 µL of the fluorescent polyacrylamide nanoparticles prepared in (1) was taken and diluted to 100 µL with 10 mM PBS buffer. 10 µL of an aqueous solution containing 0.5% EDC and 5 µL of an aqueous solution containing 0.5% NHS were added for activation at 37°C for 1 hr. The residual activator was removed by a 100 kD ultrafiltration tube, and 100 µL of 10 mM PBS buffer was added to re-suspend the nanoparticles. Then, 10 µg of streptavidin was added, and reacted at 37°C for 2 hrs. The residual streptavidin was removed by using a 150 KD ultrafiltration tube, and the nanoparticles were re-suspended in 100 µL of 10 mM PBS buffer. The ultrafiltration and resuspension steps were repeated once. The resulting second fluorescent polyacrylamide nanoparticles suspension was stored at 4°C for future use.

### 2.3. Covalent coupling of magnetic beads with capture antibody

The operation steps were the same as those in Example 1.

### 3. Experimental method

### 3.1. Determination of particle size of fluorescent polyacrylamide nanoparticles

The first and second fluorescent polyacrylamide nanoparticles were respectively 2000-fold diluted with pure water, and the particle size was measured by Marvin particle size analyzer. The particle size of the secondary fluorescent polyacrylamide particles (that is, clustered particles with the first particles as the core and the second particles as the shell) was measured as follows. Excess second particles (relative to the first particles) were allowed to react with the first particles. The particle size of the secondary particles obtained after the reaction was measured by a Malvern particle size analyzer. As a result, two peaks were obtained, and the larger peak was used as the particle size of the secondary particles.

### 3.2. Detection of cTnI antigen

(1) The cTnI antigen was diluted with fetal calf serum to give a concentration of 0, 0.01, 0.1, 0.5, 1, 5, 10, 50 and 100 pg/mL.
(2) The magnetic beads labeled with the capture antibody were diluted to 0.1 mg/mL. 50 µL was taken and added to 50 µL of the samples of various concentration obtained in (1). Then 10 µL of 100-fold diluted first fluorescent polyacrylamide nanoparticles was added and incubated at 37°C for 30 min. It was washed three times with 100 µL of washing buffer to remove the residual sample, and the supernatant was aspirated off.
(3) 10 µL of 100-fold diluted second fluorescent polyacrylamide nanoparticles was added, incubated at 37°C for 15 min, and washed with the washing buffer (4x) to remove the remaining fluorescent polyacrylamide nanoparticles. The supernatant was removed.
(4) 5 µL of the detection solution was added to re-suspend the magnetic beads, and transferred to the detection well. The magnetic beads were attracted by a magnet to the bottom of the detection well, and subjected to single molecule imaging by using a fluorescence microscope (Nikon Eclipse Ti-U). Subsequent statistical analysis of the number of single molecules was performed by single molecule counting pattern in combination with fluorescence intensity integration pattern.
(5) The detection of a series of concentrations was conducted. 3 replicates were set for each concentration point. A standard curve was plotted from the test results, and the CV% for each point was calculated.

### 4. Experimental results

### 4.1. Particle size of fluorescent polyacrylamide nanoparticles

The first and second fluorescent polyacrylamide particles both have a particle size of approximately 80 nm, and the secondary fluorescent polyacrylamide particles have a particle size of approximately 250 nm.

### 4.2. Detection of cTnI antigen

The detection result of cTnI antigen is shown in Fig. 11. It can be seen that the diluted sample with a concentration of 0.005 pg/mL can be effectively distinguished from the background. The lower detection limit in this example is calculated to be 0.002 pg/mL (that is, 2 fg/mL). It can be seen that the sensitivity of the detection method of the present application is extremely excellent.

### Example 18: Rapid detection of IL-6 antigen molecules by magnetic bead method (fluorescent polyacrylamide nanoparticles with a particle size of 220 nm)

The operation steps were the same as those in Example 14, except that the incubation time of 30 min in Step 3.2(2) of Example 14 was reduced to 3 min, and the incubation time of 15 min in Step 3.2(3) was reduced to 2 min.

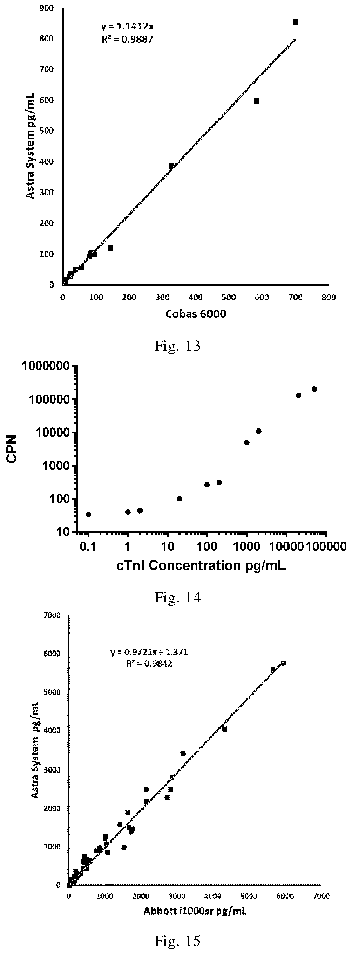

The rapid detection result of IL-6 antigen is shown in Fig. 12. It can be seen that although the detection time is reduced greatly, the diluted sample with a concentration of 1pg/mL can also be effectively distinguished from the background. The lower detection limit in Example 18 is calculated to be 1 pg/mL. In addition, 20 clinical blood samples were tested in this example. As shown in Fig. 13, the horizontal ordinate is the reference value measured by the Roche Cobas 6000 fully automatic electroluminescent device, and the longitudinal ordinate is the measured value obtained by the detection method of the present application. It can be known from the analysis of correlation that R² is 0.9887, proving that the method of the present application has a good correlation with the method used by the commercially available sophisticated device.

### Example 19: Rapid detection of cTnI antigen molecules by magnetic bead method (fluorescent polyacrylamide nanoparticles with a particle size of 220 nm)

The operation steps were the same as those in Example 15, except that the incubation time of 30 min in Step 3.2(2) of Example 15 was reduced to 3 min, and the incubation time of 15 min in Step 3.2(3) was reduced to 2 min.

The rapid detection result of cTnI antigen is shown in Fig. 14. It can be seen that although the detection time is reduced greatly, the diluted sample with a concentration of 1 pg/mL can also be effectively distinguished from the background. The lower detection limit in Example 19 is calculated to be 0.5 pg/mL. In addition, 20 clinical blood samples were tested in this example. As shown in Fig. 15, the horizontal ordinate is the reference value measured by the Abbott i1000sr fully automatic electroluminescent device, and the longitudinal ordinate is the measured value obtained by the detection method of the present application. It can be known from the analysis of correlation that R² is 0.9842, proving that the method of the present application has a good correlation with the method used by the commercially available sophisticated device.

Example 20: Detection of cTnI antigen molecules in human serum by magnetic bead method (fluorescent microspheres made of polystyrene wrapped quantum dots (hereinafter referred to as fluorescent polystyrene nanoparticles) with a particle size of 210 nm).

### 1. Reagents used in experiment

Quantum dots (Qdot 605, Thermo), polystyrene microspheres with carboxylated surface (particle size 210 nm, Hangzhou Micro-Bio)

### 2. Preparation method

### 2.1. Fluorescent polystyrene nanoparticles

(1) 0.1 g of polystyrene microspheres carboxylated on the surface were dispersed in 5 mL of 0.25% SDS. Qdot 605 and 1 mL of methylene chloride were added, and ultrasonicated at room temperature for 1 hr.
(2) The solvent was removed by rotary evaporation; and the remaining liquid was centrifuged, and the supernatant was removed The recovered solid was washed three times with ethanol and then washed 5 times with ultrapure water, and then re-suspended in 10 mL of ultrapure water and stored.

### 2.2. Covalent coupling of magnetic beads with capture antibody

The operation steps were the same as those in Example 1.

### 2.3. Covalent coupling of detection antibody and fluorescent polystyrene nanoparticles

(1) 5 µL of fluorescent polystyrene nanoparticles prepared in (2.1) were added to 40 µL of PBS buffer and ultrasonicated for 1 min.
(2) 0.01 g of EDC was dissolved in 50 µL of PBS buffer, and 0.015 g of NHS was dissolved in 150 µL of PBS buffer.
(3) The fluorescent polystyrene nanoparticles were centrifuged at 10000 rpm, and the supernatant was removed. The fluorescent polystyrene nanoparticles were re-suspended in 50 µL of PBS buffer, ultrasonicated for 1 min, added with 2.5 µL of EDC solution and 7.5 µL of NHS solution, mixed well, and reacted at 37°C for 15 min with rotation and mixing. It was centrifuged at 12000 rpm for 15 min, the supernatant was removed, and the nanoparticles were re-suspended in 50 µL of PBS buffer.
(4) 5 µg of detection antibody (Hytest 16A11) was added, , and reacted at 37°C for 2 hrs with rotation and mixing.
(5) 25 µL of Buffer D was added for blocking at 37°C for 1 hr. Then, the nanoparticles were centrifuged at 12000 rpm for 15 min and then re-suspended and preserved in 100 µL of Buffer E.

### 3. Experimental method

### Plotting of standard curve

The operation steps were the same as those in Example 1, except that fluorescent polystyrene nanoparticles bound with detection antibody was added in Step 3.1(3).

### 4. Experimental results

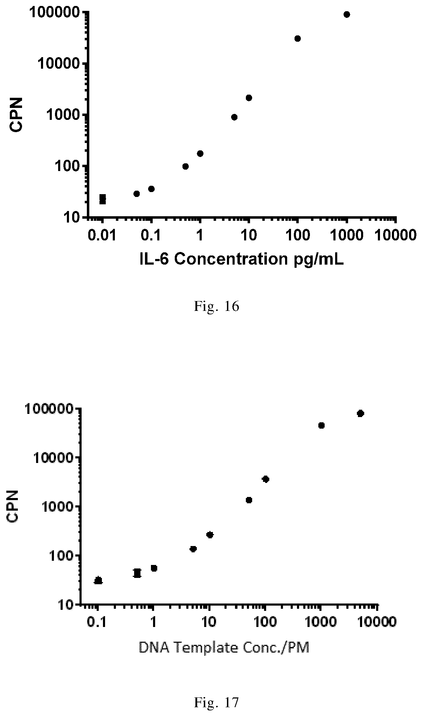

The detection results are shown in Fig. 16. It can be seen that in this example, the detection range of cTnI is 50 fg/mL - 100 pg/mL. In this range, the number of single molecule signals is in a good linear relationship with the sample concentration. The lower detection limit can reach about 50 fg/mL.

### Example 21 (corresponding to the second embodiment of the present application)

The operation steps were the same as those in Example 1, except that in Steps 3.1 and 3.2 of Example 1, the fluorescent silica nanoparticles bound with the detection antibody were reacted with a clinical sample and then magnetic beads labeled with the capture antibody were added. The results are shown in Table 1.

### Example 22: Quantitative detection of DNA molecules in buffer by glass chip method (fluorescent silica nanoparticles with a particle size of 220 nm)

### 1. Reagents used in experiment

Low-adsorption slide (Thermo), capture probe (synthesized by Sangon Biotech (Shanghai) Co., Ltd, and having a sequence shown below), detection probe (synthesized by Sangon Biotech (Shanghai) Co., Ltd, and having a sequence shown below), DNA template (synthesized by Sangon Biotech (Shanghai) Co., Ltd, and having a sequence shown below), Silane coupling agent (APTES), aqueous ammonia, ethyl orthosilicate (TEOS), fluorescein isothiocyanate (FITC), succinic anhydride, double-terminal-carboxylated polyethylene glycol, and PBS buffer,
Sequence of capture probe:
   NH₂-TTTTTTTTTTTGTGTGACATGTTCTAATATAGTCACAT
Sequence of detection probe:
   TCTGATATAATCTTGTACAGTGTGTTTTTTTTTT-NH₂
Sequence of DNA template:

CACACTGTACAAGATTATATCAGAATGTGACTATATTAGAACATGTCA
CAC

### 2. Preparation method

### 2.1. Synthesis of fluorescent silica nanoparticles and surface modification by carboxylation

The operation steps were the same as those in Example 1.

### 2.2. Surface activation of slide and modification of capture probe

(1) A low-absorption slide (Thermo) was ultrasonicated in ultrapure water for 1 hr, and dried at 70°C for 12 hrs under dust-free conditions. The surface of the slide was cleaned in a plasma cleaner, to produce highly active hydroxyl groups on the surface of the slide.
(2) The activated slide was immersed in 1% APTES and reacted at 37°C for 2 hrs to aminate the surface of the slide. The surface of the slide was washed 5 times with ultrapure water and dried at 37°C for use.
(3) 0.5% double-terminal-carboxylated polyethylene glycol was formulated, and added with 5 times the amount of EDC and 10 times the amount of NHS. After activation for 10 min, the slide obtained in (2) was immersed in the activated polyethylene glycol solution. After reaction for 15 min, the surface of the slide was washed 5 times with ultrapure water, and then blow dried with nitrogen.
(4) 300 µL of 1 µM capture probe was added dropwise to the surface of the slide. The reaction was conducted at room temperature for 1 hr, and it was washed 5 times with ultrapure water to remove unreacted capture probe.
(5) The slide bound with the capture probe was immersed in Buffer D, incubated at 37°C overnight, washed 5 times with ultrapure water, blow dried with nitrogen, and dried overnight at 50°C for use.

### 2.3. Covalent coupling of detection probe and fluorescent silica nanoparticles

(1) 10 µL of fluorescent silica nanoparticles were added to 40 µL of PBS buffer and ultrasonicated for 1 min.
(2) 0.01g of EDC was dissolved in 50 µL of PBS buffer, and 0.025g of NHS was dissolved in 150 µL of PBS buffer.
(3) The fluorescent silica nanoparticles were centrifuged at 12000 rpm, and the supernatant was removed. The fluorescent silica nanoparticles were re-suspended in 50 µL of PBS buffer, ultrasonicated for 1 min, added with 2.5 µL of EDC solution, and ultrasonicated for 1 min. Then, 7.5 µL of NHS solution was added, mixed well, and reacted at 37°C for 15 min with rotation and mixing. It was centrifuged at 12000 rpm for 15 min, the supernatant was removed, and the nanoparticles were re-suspended in 50 µL of PBS buffer.
(4) 10 µL of 10 µM detection probe was added, and reacted at 37°C for 2 hrs with rotation and mixing.
(5) 25 µL of Buffer D was added for blocking at 37°C for 1 hr. Then, it was centrifuged at 12000 rpm for 15 min and then re-suspended and preserved in 100 µL of Buffer E.

### 3. Experimental method

### Plotting of standard curve

(1) The DNA template molecules were diluted with the sample diluent to give a concentration of 0, 1, 10, 50, 100 and 1000 pM.
(2) 50 µL of the sample was added dropwise to the reaction area of the slide, and reacted at room temperature for 30 min.
(3) 50 µL of fluorescent silica nanoparticles bounded with the detection probe was added, incubated at room temperature for 30 min, and washed with the washing buffer (4x) to remove the remaining fluorescent silica nanoparticles. The supernatant was removed.
(4) Single molecule imaging was performed under a fluorescence microscope (Nikon Eclipse Ti-U). Subsequent statistical analysis of the number of single molecules was performed by single molecule counting pattern in combination with fluorescence intensity integration pattern.
(5) The detection of a series of concentrations was conducted. 6 replicates were set for each concentration point. A standard curve was plotted from the test results, and the CV% for each point was calculated.

### 4. Experimental results

The detection result is shown in Fig. 17. It can be seen that in this example, the lower detection limit of DNA template molecule is 0.5 pM, which is close to the detection sensitivity of PCR.

### Example 23: Quantitative detection of DNA molecules in buffer by magnetic bead method (fluorescent silica nanoparticles with a particle size of 220 nm)

The operation steps were the same as those in Example 22, except that the low-absorption glass slide in Example 22 was replaced by tosyl-activated M280 magnetic beads. The results are shown in Table 2.

In addition, experiments with in-situ signal enhancing nanoparticles that are respectively fluorescent particles formed of polystyrene wrapped rare earth elements (europium), fluorescent particles formed of dextran wrapped green fluorescent protein (GFP), and fluorescent particles formed of cross-linked agarose wrapped quantum dots (cadmium sulfide) were also conducted by the applicants. Specifically, the fluorescent particles formed of silica wrapped fluorescein in Example 1 were replaced by the above three types of fluorescent particles respectively. The results show that the sensitivity is also excellent (between 50 fg/mL and 5 fg/mL).

**Table 1**

| | Example 1 | Example 2 | Example 3 | Example 4 | Example 5 | Example 6 | Example 7 | Example 8 | Example 9 | Example 10 | Ex 12 |
|---|---|---|---|---|---|---|---|---|---|---|---|
| Sensitivity (expressed as the lower detection limit) | 30fg/ mL | 80fg/ mL | 100fg/ mL | 10fg/ mL | 25fg/ mL | 50fg/ mL | 16pg/ mL | 21pg/ mL | 40pg/ mL | 50pg/ mL | 35fg/ mL |
| | Example 13 | Example 14 | Example 15 | Example 16 | Example 17 | Example 20 | Example 21 | Comparative Example 1 | Comparative Example 2 | Comparative Example 3 | |
| Sensitivity | 34fg/ mL | 6fg/ mL | 8fg/ mL | 20fg/ mL | 2fg/ mL | 50fg/ mL | 45fg/ mL | Not detectable | 100 pg/mL | Not detectable | |

**Table 2**

| | Example 22 | Example 23 |
|---|---|---|
| Sensitivity (Expressed as the lower detection limit) | 0.5pM | 0.1pM |

## Claims

1. A quantitative single-molecule detection and analysis method comprising the following steps:
(1) immobilizing a capture antibody able to bind to a target molecule to a solid-phase carrier, and using the capture antibody to bind to a first site of the target molecule to capture the target molecule in a sample;
(2) adding a detection antibody that binds to a second site of the target molecule, and then adding in-situ signal enhancing nanoparticles able to directly or indirectly bind to the detection antibody; or
allowing the detection antibody to bind to the in-situ signal enhancing nanoparticles to form a complex material, and then adding the complex material;
wherein the in-situ signal enhancing nanoparticles comprise a luminescent material and a nanoparticle carrier, and have a particle size of 180-350 nm, and the luminescent material is a fluorescent material;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation,
wherein the solid-phase carrier comprises magnetic beads.

2. A quantitative single-molecule detection and analysis method comprising the following steps:
(1) allowing a detection antibody to bind to a second site of a target molecule in a sample, and then adding in-situ signal enhancing nanoparticles able to directly or indirectly bind to the detection antibody; or
allowing the in-situ signal enhancing nanoparticles to bind to the detection antibody to form a complex material, and then adding the complex material to the sample to allow the complex material to bind to the second site of the target molecule in the sample;
wherein the in-situ signal enhancing nanoparticles comprise a luminescent material and a nanoparticle carrier, and have a particle size of 180-350 nm, and the luminescent material is a fluorescent material;
(2) immobilizing a capture antibody able to bind to the target molecule to a solid-phase carrier, and then allowing the capture antibody to bind to a first site of the target molecule to capture the target molecule;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation,
wherein the solid-phase carrier comprises magnetic beads.

3. The quantitative single-molecule detection and analysis method according to claim 1 or 2, wherein the in-situ signal enhancing nanoparticles have a particle size of 200-350 nm, and further preferably 220-350 nm.

4. The quantitative single-molecule detection and analysis method according to claim 1 or 2, wherein the target molecule comprises proteins, polysaccharides or biologically active small molecules.

5. The quantitative single-molecule detection and analysis method according to claim 1 or 2, wherein in Step (4), the concentration of the target molecule in the sample is calculated by the following ways:
directly analyzing and calculating the number of bright spots formed by the in-situ signal enhancing nanoparticles in the generated image, and then directly or indirectly converting the number of bright spots into the concentration information of the target molecule in the sample; or
calculating and integrating the area of bright spots formed by the in-situ signal enhancing nanoparticles in the generated image, dividing the integrated result by an average bright spot area formed by each in-situ signal enhancing nanoparticle in average to obtain the approximate number of the in-situ signal enhancing nanoparticles by conversion, and then converting the number into the concentration information of the target molecule in the sample.

6. The quantitative single-molecule detection and analysis method according to any one of claims 1 to 5, wherein the luminescent material in the in-situ signal enhancing nanoparticles is fluorescein or quantum dots; the nanoparticle carrier in the in-situ signal enhancing nanoparticles comprises silica, polyacrylamide, polystyrene or polymethyl methacrylate; and in Step (4), the single molecule counting pattern is used in combination with the fluorescence intensity integration pattern.

7. A quantitative single-molecule detection and analysis method comprising the following steps:
(1) immobilizing a capture probe able to bind to a target molecule to a solid-phase carrier, wherein the capture probe is complementary to a first sequence of the target molecule; and capturing the target molecule in a sample by the capture probe;
(2) adding a detection probe that is complementary to a second sequence of the target molecule, to form a three-strand hybrid structure of capture probe-target molecule-detection probe, and then adding in-situ signal enhancing nanoparticles able to directly or indirectly bind to the detection probe; or
allowing the detection probe to bind to the in-situ signal enhancing nanoparticles to form a complex material, and then adding the complex material;
wherein the in-situ signal enhancing nanoparticles comprise a luminescent material and a nanoparticle carrier, and have a particle size of 180-350 nm, and the luminescent material is a fluorescent material;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation,
wherein the solid-phase carrier comprises magnetic beads.

8. A quantitative single-molecule detection and analysis method comprising the following steps:
(1) allowing a detection probe to be complementary to a second sequence of a target molecule in a sample, and then adding in-situ signal enhancing nanoparticles directly or indirectly binding to the detection probe; or
allowing the in-situ signal enhancing nanoparticles to bind to the detection probe to form a complex material in advance, and then allowing the complex material to be complementary to a second sequence of the target molecule in the sample;
wherein the in-situ signal enhancing nanoparticles comprise a luminescent material and a nanoparticle carrier, and have a particle size of 180-350 nm, and the luminescent material is a fluorescent material;
(2) immobilizing a capture probe able to bind to the target molecule to a solid-phase carrier, and then allowing the capture probe to be complementary to a first sequence of the target molecule to capture the target molecule;
(3) detecting an optical signal emitted by the in-situ signal enhancing nanoparticles by an optical imaging device; and
(4) calculating the number of the in-situ signal enhancing nanoparticles, and obtaining the concentration information of the target molecule in the sample after further calculation,
wherein the solid-phase carrier comprises magnetic beads.

9. The quantitative single-molecule detection and analysis method according to claim 7 or 8, wherein the in-situ signal enhancing nanoparticles have a particle size of 200-350 nm, and further preferably 220-350 nm.

10. The quantitative single-molecule detection and analysis method according to claim 7 or 8, wherein the target molecule comprises DNA or RNA.

11. The quantitative single-molecule detection and analysis method according to claim 7 or 8, wherein in Step (4), the concentration of the target molecule in the sample is calculated by the following ways:
directly analyzing and calculating the number of bright spots formed by the in-situ signal enhancing nanoparticles in the generated image, and then directly or indirectly converting the number of bright spots into the concentration information of the target molecule in the sample; or
calculating and integrating the area of bright spots formed by the in-situ signal enhancing nanoparticles in the generated image, dividing the integrated result by an average bright spot area formed by each in-situ signal enhancing nanoparticle in average to obtain the approximate number of the in-situ signal enhancing nanoparticles by conversion, and then converting the number into the concentration information of the target molecule in the sample.

12. A quantitative single-molecule detection system, comprising:
(1) a detection reagent, comprising:
(a) a capture antibody able to bind to a first site of a target molecule to capture the target molecule in a sample; (b) a detection antibody able to bind to a second site of the target molecule and able to bind to in-situ signal enhancing nanoparticles; and (c) the in-situ signal enhancing nanoparticles comprising a luminescent material and a nanoparticle carrier and having a particle size of 180-350 nm, and the luminescent material is a fluorescent material; (d) a solid-phase carrier, the solid-phase carrier comprises magnetic beads and is able to immobilize the capture probe; or
(a) a capture probe able to bind to a first sequence of a target molecule to capture the target molecule in a sample; (b) a detection probe able to bind to a second sequence of the target molecule to form a three-strand hybrid structure of capture probe-target molecule-detection probe; and (c) in-situ signal enhancing nanoparticles able to bind to the detection probe, comprising a luminescent material and a nanoparticle carrier and having a particle size of 180-350 nm, and the luminescent material is a fluorescent material; (d) a solid-phase carrier, the solid-phase carrier comprises magnetic beads and is able to immobilize the capture probe; and
(2) an optical imaging device comprising an excitation light source and an optical signal acquisition unit.

13. The quantitative single-molecule detection and analysis system according to claim 12, wherein the in-situ signal enhancing nanoparticles have a particle size of 200-350 nm, and further preferably 220-350 nm.

14. The quantitative single-molecule detection and analysis system according to claim 12, wherein the optical imaging device further comprises a data processing unit having a data acquisition module and a data processing module, wherein the data acquisition module is configured to receive an optical signal captured by a light sensing element and convert the optical signal into a digital signal; the data processing module is configured for conversion of the digital signal and formation and processing of an optical image.

## Patentansprüche

1. Quantitatives Verfahren zum Nachweisen und Analysieren eines einzelnen Moleküls, umfassend die folgenden Schritte:
(1) Immobilisieren eines Fänger-Antikörpers, der in der Lage ist, an ein Zielmolekül zu binden, auf einem Festphasenträger und Verwenden des Fänger-Antikörpers zum Binden an eine erste Stelle des Zielmoleküls, um das Zielmolekül in einer Probe einzufangen;
(2) Hinzufügen eines Nachweis-Antikörpers, der an eine zweite Stelle des Zielmoleküls bindet, und anschließendes Hinzufügen von signalverstärkenden In-situ-Nanopartikeln, die in der Lage sind, direkt oder indirekt an den Nachweis-Antikörper zu binden; oder
Ermöglichen der Bindung des Nachweis-Antikörpers an die signalverstärkenden In-situ-Nanopartikel, um ein komplexes Material zu bilden, und anschließendes Hinzufügen des komplexen Materials;
wobei die signalverstärkenden In-situ-Nanopartikel ein lumineszierendes Material und einen Nanopartikelträger umfassen und eine Partikelgröße von 180-350 nm aufweisen, und das lumineszierende Material ein fluoreszierendes Material ist;
(3) Nachweisen eines von den signalverstärkenden In-situ-Nanopartikeln ausgestrahlten optischen Signals mittels einer optischen Bildgebungsvorrichtung; und
(4) Berechnen der Anzahl der signalverstärkenden In-situ-Nanopartikel und Erhalten der Konzentrationsinformation des Zielmoleküls in der Probe nach weiterer Berechnung,
wobei der Festphasenträger magnetische Kügelchen umfasst.

2. Quantitatives Verfahren zum Nachweisen und Analysieren eines einzelnen Moleküls, umfassend die folgenden Schritte:
(1) Ermöglichen der Bindung eines Nachweis-Antikörpers an eine zweite Stelle eines Zielmoleküls in einer Probe und anschließendes Hinzufügen von signalverstärkenden In-situ-Nanopartikeln, die in der Lage sind, direkt oder indirekt an den Nachweis-Antikörper zu binden; oder
Ermöglichen der Bindung der signalverstärkenden In-situ-Nanopartikel an den Nachweis-Antikörper, um ein komplexes Material zu bilden, und anschließendes Hinzufügen des komplexen Materials zur Probe, um die Bindung des komplexen Materials an die zweite Stelle des Zielmoleküls in der Probe zu ermöglichen;
wobei die signalverstärkenden In-situ-Nanopartikel ein lumineszierendes Material und einen Nanopartikelträger umfassen und eine Partikelgröße von 180-350 nm aufweisen, und das lumineszierende Material ein fluoreszierendes Material ist;
(2) Immobilisieren eines Fänger-Antikörpers, das in der Lage ist, an das Zielmolekül zu binden, auf einem Festphasenträger und anschließendes Ermöglichen der Bindung des Fänger-Antikörpers an eine erste Stelle des Zielmoleküls, um das Zielmolekül einzufangen;
(3) Nachweisen eines von den signalverstärkenden In-situ-Nanopartikeln ausgestrahlten optischen Signals mittels einer optischen Bildgebungsvorrichtung; und
(4) Berechnen der Anzahl der signalverstärkenden In-situ-Nanopartikel und Erhalten der Konzentrationsinformation des Zielmoleküls in der Probe nach weiterer Berechnung,
wobei der Festphasenträger magnetische Kügelchen umfasst.

3. Quantitatives Verfahren zum Nachweisen und Analysieren eines einzelnen Moleküls nach Anspruch 1 oder 2, wobei die signalverstärkenden In-situ-Nanopartikel eine Partikelgröße von 200-350 nm und ferner bevorzugt von 220-350 nm aufweisen.

4. Quantitatives Verfahren zum Nachweisen und Analysieren eines einzelnen Moleküls nach Anspruch 1 oder 2, wobei das Zielmolekül Proteine, Polysaccharide oder biologisch aktive kleine Moleküle umfasst.

5. Quantitatives Verfahren zum Nachweisen und Analysieren eines einzelnen Moleküls nach Anspruch 1 oder 2, wobei in Schritt (4) die Konzentration des Zielmoleküls in der Probe auf folgende Weise berechnet wird:
direktes Analysieren und Berechnen der Anzahl heller Flecken, die durch die signalverstärkenden In-situ-Nanopartikel im erzeugten Bild gebildet werden, und anschließendes direktes oder indirektes Umwandeln der Anzahl der hellen Flecken in die Konzentrationsinformation des Zielmoleküls in der Probe; oder
Berechnen und Integrieren der Fläche der hellen Flecken, die durch die signalverstärkenden In-situ-Nanopartikel im erzeugten Bild gebildet werden, Teilen des integrierten Ergebnisses durch eine durchschnittliche Fläche der hellen Flecken, die durchschnittlich von jedem signalverstärkenden In-situ-Nanopartikel gebildet wird, um die ungefähre Anzahl der signalverstärkenden In-situ-Nanopartikel durch Umwandlung zu erhalten, und anschließendes Umwandeln der Anzahl in die Konzentrationsinformation des Zielmoleküls in der Probe.

6. Quantitatives Verfahren zum Nachweisen und Analysieren eines einzelnen Moleküls nach einem der Ansprüche 1 bis 5, wobei das lumineszierende Material in den signalverstärkenden In-situ-Nanopartikeln Fluorescein oder Quantenpunkte ist; der Nanopartikelträger in den signalverstärkenden In-situ-Nanopartikeln Siliciumdioxid, Polyacrylamid, Polystyrol oder Polymethylmethacrylat umfasst; und in Schritt (4) das Einzelmolekül-Zählmuster in Kombination mit dem Fluoreszenzintensitäts-Integrationsverfahren verwendet wird.

7. Quantitatives Verfahren zum Nachweisen und Analysieren eines einzelnen Moleküls, umfassend die folgenden Schritte:
(1) Immobilisieren einer Fängersonde, die in der Lage ist, an ein Zielmolekül zu binden, auf einem Festphasenträger, wobei die Fängersonde komplementär zu einer ersten Sequenz des Zielmoleküls ist; und Einfangen des Zielmoleküls in einer Probe mittels der Fängersonde;
(2) Hinzufügen einer Nachweissonde, die komplementär zu einer zweiten Sequenz des Zielmoleküls ist, um eine dreisträngige Hybridstruktur aus Fängersonde-Zielmolekül-Nachweissonde zu bilden, und anschließendes Hinzufügen von signalverstärkenden In-situ-Nanopartikeln, die in der Lage sind, direkt oder indirekt an die Nachweissonde zu binden; oder
Ermöglichen der Bindung der Nachweissonde an die signalverstärkenden In-situ-Nanopartikel, um ein komplexes Material zu bilden, und anschließendes Hinzufügen des komplexen Materials;
wobei die signalverstärkenden In-situ-Nanopartikel ein lumineszierendes Material und einen Nanopartikelträger umfassen und eine Partikelgröße von 180-350 nm aufweisen, wobei das lumineszierende Material ein fluoreszierendes Material ist;
(3) Nachweisen eines von den signalverstärkenden In-situ-Nanopartikeln ausgestrahlten optischen Signals mittels einer optischen Bildgebungsvorrichtung; und
(4) Berechnen der Anzahl der signalverstärkenden In-situ-Nanopartikel und Erhalten der Konzentrationsinformation des Zielmoleküls in der Probe nach weiterer Berechnung,
wobei der Festphasenträger magnetische Kügelchen umfasst.

8. Quantitatives Verfahren zum Nachweisen und Analysieren eines einzelnen Moleküls, umfassend die folgenden Schritte:
(1) Ermöglichen der Komplementarität einer Nachweissonde zu einer zweiten Sequenz eines Zielmoleküls in einer Probe und anschließendes Hinzufügen von signalverstärkenden In-situ-Nanopartikeln, die direkt oder indirekt an die Nachweissonde binden; oder
Ermöglichen der Bindung der signalverstärkenden In-situ-Nanopartikel an die Nachweissonde, um ein komplexes Material im Voraus zu bilden, und anschließendes Ermöglichen der Komplementarität des komplexen Materials zu einer zweiten Sequenz des Zielmoleküls in der Probe;
wobei die signalverstärkenden In-situ-Nanopartikel ein lumineszierendes Material und einen Nanopartikelträger umfassen und eine Partikelgröße von 180-350 nm aufweisen, und das lumineszierende Material ein fluoreszierendes Material ist;
(2) Immobilisieren einer Fängersonde, die in der Lage ist, an das Zielmolekül zu binden, auf einem Festphasenträger und anschließendes Ermöglichen der Komplementarität der Fängersonde zu einer ersten Sequenz des Zielmoleküls, um das Zielmolekül einzufangen;
(3) Nachweisen eines von den signalverstärkenden In-situ-Nanopartikeln ausgestrahlten optischen Signals mittels einer optischen Bildgebungsvorrichtung; und
(4) Berechnen der Anzahl der signalverstärkenden In-situ-Nanopartikel und Erhalten der Konzentrationsinformation des Zielmoleküls in der Probe nach weiterer Berechnung,
wobei der Festphasenträger magnetische Kügelchen umfasst.

9. Quantitatives Verfahren zum Nachweisen und Analysieren eines einzelnen Moleküls nach Anspruch 7 oder 8, wobei die signalverstärkenden In-situ-Nanopartikel eine Partikelgröße von 200-350 nm und ferner bevorzugt von 220-350 nm aufweisen.

10. Quantitatives Verfahren zum Nachweisen und Analysieren eines einzelnen Moleküls nach Anspruch 7 oder 8, wobei das Zielmolekül DNA oder RNA umfasst.

11. Quantitatives Verfahren zum Nachweisen und Analysieren eines einzelnen Moleküls nach Anspruch 7 oder 8, wobei in Schritt (4) die Konzentration des Zielmoleküls in der Probe auf folgende Weise berechnet wird:
direktes Analysieren und Berechnen der Anzahl heller Flecken, die durch die signalverstärkenden In-situ-Nanopartikel im erzeugten Bild gebildet werden, und anschließendes direktes oder indirektes Umwandeln der Anzahl der hellen Flecken in die Konzentrationsinformation des Zielmoleküls in der Probe; oder
Berechnen und Integrieren der Fläche der hellen Flecken, die durch die signalverstärkenden In-situ-Nanopartikel im erzeugten Bild gebildet werden, Teilen des integrierten Ergebnisses durch eine durchschnittliche Fläche der hellen Flecken, die durchschnittlich von jedem signalverstärkenden In-situ-Nanopartikel gebildet wird, um die ungefähre Anzahl der signalverstärkenden In-situ-Nanopartikel durch Umwandlung zu erhalten, und anschließendes Umwandeln der Anzahl in die Konzentrationsinformation des Zielmoleküls in der Probe.

12. Quantitatives System zum Nachweisen eines einzelnen Moleküls, umfassend:
(1) ein Nachweisreagenz, umfassend:
(a) einen Fänger-Antikörper, der in der Lage ist, an eine erste Stelle eines Zielmoleküls zu binden, um das Zielmolekül in einer Probe einzufangen; (b) einen Nachweis-Antikörper, der in der Lage ist, an eine zweite Stelle des Zielmoleküls zu binden, und der in der Lage ist, an signalverstärkende In-situ-Nanopartikel zu binden; und (c) wobei die signalverstärkenden In-situ-Nanopartikel ein lumineszierendes Material und einen Nanopartikelträger umfassen und eine Partikelgröße von 180-350 nm aufweisen, und das lumineszierende Material ein fluoreszierendes Material ist; (d) einen Festphasenträger, wobei der Festphasenträger magnetische Kügelchen umfasst und in der Lage ist, die Fängersonde zu immobilisieren; oder
(a) eine Fängersonde, die in der Lage ist, an eine erste Sequenz eines Zielmoleküls zu binden, um das Zielmolekül in einer Probe einzufangen; (b) eine Nachweissonde, die in der Lage ist, an eine zweite Sequenz des Zielmoleküls zu binden, um eine dreisträngige Hybridstruktur aus Fängersonde-Zielmolekül-Nachweissonde zu bilden; und (c) signalverstärkende In-situ-Nanopartikel, die in der Lage sind, an die Nachweissonde zu binden, ein lumineszierendes Material und einen Nanopartikelträger umfassen und eine Partikelgröße von 180-350 nm aufweisen, und das lumineszierende Material ein fluoreszierendes Material ist; (d) einen Festphasenträger, wobei der Festphasenträger magnetische Kügelchen umfasst und in der Lage ist, die Fängersonde zu immobilisieren; und
(2) eine optische Bildgebungsvorrichtung, die eine Anregungslichtquelle und eine optische Signalerfassungseinheit umfasst.

13. Quantitatives System zum Nachweisen und Analysieren eines einzelnen Moleküls nach Anspruch 12, wobei die signalverstärkenden In-situ-Nanopartikel eine Partikelgröße von 200-350 nm und ferner bevorzugt von 220-350 nm aufweisen.

14. Quantitatives System zum Nachweisen und Analysieren eines einzelnen Moleküls nach Anspruch 12, wobei die optische Bildgebungsvorrichtung ferner eine Datenverarbeitungseinheit umfasst, die ein Datenerfassungsmodul und ein Datenverarbeitungsmodul aufweist, wobei das Datenerfassungsmodul dazu konfiguriert ist, ein durch ein lichtempfindliches Element eingefangenes optisches Signal zu empfangen und das optische Signal in ein digitales Signal umzuwandeln; wobei das Datenverarbeitungsmodul dazu konfiguriert ist, das digitale Signal umzuwandeln und ein optisches Bild zu bilden und zu verarbeiten.

## Revendications

1. Procédé quantitatif de détection et d'analyse d'une unique molécule comprenant les étapes suivantes :
(1) immobilisation d'un anticorps de capture apte à se lier à une molécule cible sur un support en phase solide, et utilisation de l'anticorps de capture pour se lier à un premier site de la molécule cible afin de capturer la molécule cible dans un échantillon ;
(2) ajout d'un anticorps de détection se liant à un second site de la molécule cible, et puis ajout de nanoparticules de renforcement de signal in-situ aptes à se lier directement ou indirectement à l'anticorps de détection ; ou
autorisation de la liaison de l'anticorps de détection aux nanoparticules de renforcement de signal in-situ pour former un matériau complexe, et puis ajout du matériau complexe ;
les nanoparticules de renforcement de signal in-situ comprenant un matériau luminescent et un support nanoparticulaire, et présentant une taille de particule de 180 à 350 nm, et le matériau luminescent étant un matériau fluorescent ;
(3) détection d'un signal optique émis par les nanoparticules de renforcement de signal in-situ au moyen d'un dispositif d'imagerie optique ; et
(4) calcul du nombre de nanoparticules de renforcement de signal in-situ, et obtention de l'information de concentration de la molécule cible dans l'échantillon après calcul supplémentaire,
le support en phase solide comprenant des billes magnétiques.

2. Procédé quantitatif de détection et d'analyse d'une unique molécule comprenant les étapes suivantes :
(1) autorisation de la liaison d'un anticorps de détection à un second site d'une molécule cible dans un échantillon, et puis ajout de nanoparticules de renforcement de signal in-situ aptes à se lier directement ou indirectement à l'anticorps de détection ; ou
autorisation de la liaison des nanoparticules de renforcement de signal in-situ à l'anticorps de détection pour former un matériau complexe, et puis ajout du matériau complexe à l'échantillon pour permettre la liaison du matériau complexe au second site de la molécule cible dans l'échantillon ;
les nanoparticules de renforcement de signal in-situ comprenant un matériau luminescent et un support nanoparticulaire, et présentant une taille de particule de 180 à 350 nm, le matériau luminescent étant un matériau fluorescent ;
(2) immobilisation d'un anticorps de capture apte à se lier à la molécule cible sur un support en phase solide, et puis autorisation de la liaison de l'anticorps de capture à un premier site de la molécule cible pour capturer la molécule cible ;
(3) détection d'un signal optique émis par les nanoparticules de renforcement de signal in-situ au moyen d'un dispositif d'imagerie optique ; et
(4) calcul du nombre de nanoparticules de renforcement de signal in-situ, et obtention de l'information de concentration de la molécule cible dans l'échantillon après calcul supplémentaire,
le support en phase solide comprenant des billes magnétiques.

3. Procédé quantitatif de détection et d'analyse d'une unique molécule selon la revendication 1 ou 2, dans lequel les nanoparticules de renforcement de signal in-situ présentent une taille de particule de 200 à 350 nm, et de préférence de 220 à 350 nm.

4. Procédé quantitatif de détection et d'analyse d'une unique molécule selon la revendication 1 ou 2, dans lequel la molécule cible comprend des protéines, des polysaccharides ou de petites molécules biologiquement actives.

5. Procédé quantitatif de détection et d'analyse d'une unique molécule selon la revendication 1 ou 2, dans lequel à l'étape (4), la concentration de la molécule cible dans l'échantillon est calculée selon les modalités suivantes :
analyse directe et calcul du nombre de points lumineux formés par les nanoparticules de renforcement de signal in-situ dans l'image générée, et puis conversion directe ou indirecte du nombre de points lumineux en information de concentration de la molécule cible dans l'échantillon ; ou
calcul et intégration de la surface des points lumineux formés par les nanoparticules de renforcement de signal in-situ dans l'image générée, division du résultat intégré par une surface moyenne de point lumineux formée en moyenne par chaque nanoparticule de renforcement de signal in-situ pour obtenir par conversion le nombre approximatif des nanoparticules de renforcement de signal in-situ, et puis conversion du nombre en information de concentration de la molécule cible dans l'échantillon.

6. Procédé quantitatif de détection et d'analyse d'une unique molécule selon l'une quelconque des revendications 1 à 5, dans lequel le matériau luminescent des nanoparticules de renforcement de signal in-situ est la fluorescéine ou des points quantiques ; le support nanoparticulaire dans les nanoparticules de renforcement de signal in-situ comprend de la silice, du polyacrylamide, du polystyrène ou du polyméthacrylate de méthyle ; et à l'étape (4), le mode de comptage de molécule unique est utilisé en combinaison avec le mode d'intégration d'intensité de fluorescence.

7. Procédé quantitatif de détection et d'analyse d'une unique molécule comprenant les étapes suivantes :
(1) immobilisation d'une sonde de capture apte à se lier à une molécule cible sur un support en phase solide, la sonde de capture étant complémentaire d'une première séquence de la molécule cible ; et capture de la molécule cible dans un échantillon par la sonde de capture ;
(2) ajout d'une sonde de détection complémentaire d'une seconde séquence de la molécule cible, pour former une structure hybride à trois brins de sonde de capture-molécule cible-sonde de détection, et puis ajout de nanoparticules de renforcement de signal in-situ aptes à se lier directement ou indirectement à la sonde de détection ; ou
autorisation de la liaison de la sonde de détection aux nanoparticules de renforcement de signal in-situ pour former un matériau complexe, et puis ajout du matériau complexe ;
les nanoparticules de renforcement de signal in-situ comprenant un matériau luminescent et un support nanoparticulaire, et présentant une taille de particule de 180 à 350 nm, et le matériau luminescent étant un matériau fluorescent ;
(3) détection d'un signal optique émis par les nanoparticules de renforcement de signal in-situ au moyen d'un dispositif d'imagerie optique ; et
(4) calcul du nombre de nanoparticules de renforcement de signal in-situ, et obtention de l'information de concentration de la molécule cible dans l'échantillon après calcul supplémentaire,
le support en phase solide comprenant des billes magnétiques.

8. Procédé quantitatif de détection et d'analyse d'une unique molécule comprenant les étapes suivantes :
(1) autorisation de la complémentarité d'une sonde de détection à une seconde séquence d'une molécule cible dans un échantillon, puis ajout de nanoparticules de renforcement de signal in-situ se liant directement ou indirectement à la sonde de détection ; ou
autorisation de la liaison des nanoparticules de renforcement de signal in-situ à la sonde de détection pour former un matériau complexe à l'avance, puis autorisation de la complémentarité du matériau complexe à une seconde séquence de la molécule cible dans l'échantillon ;
les nanoparticules de renforcement de signal in-situ comprenant un matériau luminescent et un support nanoparticulaire, et présentant une taille de particule de 180 à 350 nm, le matériau luminescent étant un matériau fluorescent ;
(2) immobilisation d'une sonde de capture apte à se lier à la molécule cible sur un support en phase solide, et puis autorisation de la complémentarité de la sonde de capture à une première séquence de la molécule cible pour capturer la molécule cible ;
(3) détection d'un signal optique émis par les nanoparticules de renforcement de signal in-situ au moyen d'un dispositif d'imagerie optique ; et
(4) calcul du nombre de nanoparticules de renforcement de signal in-situ, et obtention de l'information de concentration de la molécule cible dans l'échantillon après calcul supplémentaire,
le support en phase solide comprenant des billes magnétiques.

9. Procédé quantitatif de détection et d'analyse d'une unique molécule selon la revendication 7 ou 8, dans lequel les nanoparticules de renforcement de signal in-situ présentent une taille de particule de 200 à 350 nm, et de préférence de 220 à 350 nm.

10. Procédé quantitatif de détection et d'analyse d'une unique molécule selon la revendication 7 ou 8, dans lequel la molécule cible comprend de l'ADN ou de l'ARN.

11. Procédé quantitatif de détection et d'analyse d'une unique molécule selon la revendication 7 ou 8, dans lequel à l'étape (4), la concentration de la molécule cible dans l'échantillon est calculée selon les modalités suivantes :
analyse directe et calcul du nombre de points lumineux formés par les nanoparticules de renforcement de signal in-situ dans l'image générée, puis conversion directe ou indirecte du nombre de points lumineux en information de concentration de la molécule cible dans l'échantillon ; ou
calcul et intégration de la surface des points lumineux formés par les nanoparticules de renforcement de signal in-situ dans l'image générée, division du résultat intégré par une surface moyenne de point lumineux formée en moyenne par chaque nanoparticule de renforcement de signal in-situ pour obtenir par conversion le nombre approximatif des nanoparticules de renforcement de signal in-situ, et puis conversion du nombre en information de concentration de la molécule cible dans l'échantillon.

12. Système quantitatif de détection d'une unique molécule, comprenant :
(1) un réactif de détection, comprenant :
(a) un anticorps de capture apte à se lier à un premier site d'une molécule cible pour capturer la molécule cible dans un échantillon ; une sonde de détection apte à se lier à une seconde séquence de la molécule cible pour former une structure hybride à trois brins de sonde de capture-molécule cible-sonde de détection ; et (c) les nanoparticules de renforcement de signal in-situ comprenant un matériau luminescent et un support nanoparticulaire et présentant une taille de particule de 180 à 350 nm, le matériau luminescent étant un matériau fluorescent ; (d) un support en phase solide, le support en phase solide comprenant des billes magnétiques et étant apte à immobiliser la sonde de capture ; ou
(a) une sonde de capture apte à se lier à une première séquence d'une molécule cible pour capturer la molécule cible dans un échantillon ; (b) une sonde de détection apte à se lier à une seconde séquence de la molécule cible pour former une structure hybride à trois brins sonde de capture-molécule cible-sonde de détection ; et (c) des nanoparticules de renforcement de signal in-situ aptes à se lier à la sonde de détection, comprenant un matériau luminescent et un support nanoparticulaire et présentant une taille de particule de 180 à 350 nm, et le matériau luminescent étant un matériau fluorescent ; (d) un support en phase solide, le support en phase solide comprenant des billes magnétiques et étant apte à immobiliser la sonde de capture ; et
(2) un dispositif d'imagerie optique comprenant une source lumineuse d'excitation et une unité d'acquisition de signal optique.

13. Système quantitatif de détection et d'analyse d'une unique molécule selon la revendication 12, dans lequel les nanoparticules de renforcement de signal in-situ présentent une taille de particule de 200 à 350 nm, et de préférence de 220 à 350 nm.

14. Système quantitatif de détection et d'analyse d'une unique molécule selon la revendication 12, dans lequel le dispositif d'imagerie optique comprend en outre une unité de traitement de données comprenant un module d'acquisition de données et un module de traitement de données, le module d'acquisition de données étant configuré pour recevoir un signal optique capturé par un élément de détection de lumière et convertir le signal optique en signal numérique ; le module de traitement de données étant configuré pour la conversion du signal numérique ainsi que la formation et le traitement d'une image optique.
